# FASCICULE DE BREVET EUROPEEN

(11) **EP 1 001 944 B1**
(45) Date de publication et mention de la délivrance du brevet: **01.10.2003**
(21) Numéro de dépôt: 98939676.7
(22) Date de dépôt: 15.07.1998
(51) Int. Cl.: C07D 241/24, C07D 241/12, C07D 213/82, C07D 213/81, C07D 213/38, A61K 31/44, A61K 31/495, C07D 213/75, C07D 213/30

(54) **DERIVES DE PYRAZINE, LEUR PREPARATION ET LES MEDICAMENTS LES CONTENANT**
PYRAZIN-DERIVATE, VERFAHREN ZU IHRER HERSTELLUNG UND DIESE ENTHALTENDE ARZNEIMITTEL
PYRAZINE DERIVATIVES, PREPARATION AND MEDICINES CONTAINING THEM

(30) Priorité: 17.07.1997 FR 9709058
(43) Date de publication de la demande: 24.05.2000
(73) Titulaire: Aventis Pharma S.A., 92160 Antony (FR)
(72) Inventeur: BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Créteil (FR); MIGNANI, Serge, F-92290 Châtenay-Malabry (FR)
(86) Numéro de dépôt international: FR9801542
(87) Numéro de publication internationale: WO99003844

(56) Documents cités:
- EP-A- 0 010 896
- EP-A- 0 463 592
- DE-A- 3 703 959
- P.D.J. GROOTENHUIS ET AL.: "Complexes of macromolecular polyethers and neutral guest molecules: a systematic approach to the complexation of water molecules by 2,6-pyridinium crown ethers" JOURNAL OF THE AMERICAN CHEMICAL SOCIETY., vol. 108, no. 4, 1986, pages 780-788, XP002078185 Washington DC US
- CHEMICAL ABSTRACTS, vol. 111, no. 21, 20 novembre 1989 Columbus, Ohio, US; abstract no. 194802s, TAGUCHI, HIROAKI ET AL: "Preparation of heterocyclic diamides as allergy inhibitors" page 779; XP002078186 & JP 01 117863 A (TOYOBO CO., LTD., JAPAN)
- KUMAR S ET AL: "Synthetic Ionophores Part 14: Effect of Pyridine and Thioether Ligating Units on AgSelectivity in 18-Membered Diamide-Diester Macrocycles<1" TETRAHEDRON., vol. 52, no. 42, 14 octobre 1996, page 13483-13492 XP004105323 OXFORD GB

## Description

La présente invention concerne des médicaments contenant en tant que principe actif un composé de formule : 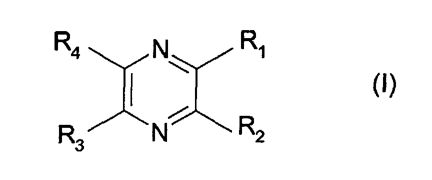 un stéréoisomère ou un sel d'un tel composé avec un acide minéral ou organique, les nouveaux composés de formule (I) et leur procédé de préparation.

Dans la formule (I)
soit R représente un atome d'azote, R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ dans lequel n est égal à 0, 1, 2, 3 ou 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ dans lequel y est égal à 0, 1, 2, 3 ou 4, -CH₂-N(CH(CH₂OH)₂)₂ ou -CH₂-N(CH₂-CH(CH₂OH)₂)₂ et l'un des substituants R₃ ou R₄ représente un atome d'hydrogène et l'autre est identique à R₁ et R₂ représente un atome d'hydrogène,
R₅ représente un atome d'hydrogène ou un radical alkyle,
R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ ou -CH(CH₂OH)-(CHOH)ₓ-CH₂OH dans lequel x est égal à 1, 2, 3 ou 4,
alk représente un radical alkyle.

Dans les définitions qui précédent et celles qui suivent, les radicaux alkyle contiennent 1 à 6 atomes de carbone en chaîne droite ou ramifiée.

Les composés de formule (I) comportant des atomes de carbone asymétriques, présentent des formes stéréoisomères. Ces stéréoisomères font partie de l'invention.

Les médicaments préférés sont ceux qui contiennent un composé de formule (I) choisi parmi les composés suivants :
-N,N'-bis-(2-hydroxyéthyl)-pyrazine-2,5-dicarboxamide,
-N,N'-bis-(2-hydroxyéthyl)-pyrazine-2,6-dicarboxamide,
-N-méthyl-N-(2-hydroxyéthyl)-N'-méthyl-N'(2-hydroxyéthyl)-pyrazine-2,5-dicarboxamide,
-N-méthyl-N-(2-hydroxyéthyl)-N'-méthyl-N'(2-hydroxyéthyl)-pyrazine-2,6-dicarboxamide,
-N,N,N',N'-tétrakis(2-hydroxyéthyl)-pyrazine-2,5-dicarboxamide,
-N,N,N',N'-tétrakis(2-hydroxyéthyl)-pyrazine-2,6-dicarboxamide,
-N,N'-bis-(2,3-dihydroxyprop-1-yl)-pyrazine-2,5-dicarboxamide,
-N,N'-bis-(2,3-dihydroxyprop-1-yl]-pyrazine-2,6-dicarboxamide,
-N-méthyl-N-(2,3-dihydroxyprop-1-yl)-N'-méthyl-N'-(2,3-dihydroxyprop-1-yl)-pyrazine-2,5-dicarboxamide,
-N-méthyl-N-(2,3-dihydroxyprop-1-yl)-N'-méthyl-N'-(-2,3-dihydroxyprop-1-yl)-pyrazine-2,6-dicarboxamide,
-N,N,N',N'-tétrakis-(2,3-dihydroxyprop-1-yl)-pyrazine-2,5-dicarboxamide,
-N,N,N',N'-tétrakis-(2,3-dihydroxyprop-1-yl]-pyrazine-2,6-dicarboxamide,
-N,N'-bis-(1,3-dihydroxyprop-2-yl)-pyrazine-2,5-dicarboxamide,
-N,N'-bis-(1,3-dihydroxyprop-2-yl]-pyrazine-2,6-dicarboxamide,
-N-méthyl-N-(1,3-dihydroxyprop-2-yl)-N'-méthyl-N'-(1,3-dihydroxyprop-2-yl)-pyrazine-2,5-dicarboxamide,
-N-méthyl-N-(1,3-dihydroxyprop-2-yl)-N'-méthyl-N'-(1,3-dihydroxyprop-2-yl)-pyrazine-2,6-dicarboxamide,
-N,N,N',N'-tétrakis-(1,3-dihydroxyprop-2-yl)-pyrazine-2,5-dicarboxamide,
-N,N,N',N'-tétrakis-(1,3-dihydroxyprop-2-yl)-pyrazine-2,6-dicarboxamide,
-N,N'-bis-(2,3,4-trihydroxybut-1-yl)-pyrazine-2,5-dicarboxamide,
-N,N'-bis-(2,3,4-trihydroxybut-1-yl)-pyrazine-2,6-dicarboxamide,
-N-méthyl-N-(2,3,4-trihydroxybut-1-yl)-N'-méthyl-N'-(2,3,4-trihydroxybut-1-yl)-pyrazine-2,5-dicarboxamide,
-N-méthyl-N-(2,3,4-trihydroxybut-1-yl)-N'-méthyl-N'-(2,3,4-trihydroxybut-1-yl)-pyrazine-2,6-dicarboxamide,
-N,N'-bis-(1,3,4-trihydroxybut-2-yl)-pyrazine-2, 5-dicarboxamide,
-N,N'-bis-(1,3,4-trihydroxybut-2-yl)-pyrazine-2,6-dicarboxamide,
-N-méthyl-N-(1,3,4-trihydroxybut-2-yl)-N'-méthyl-N'-(1,3,4-trihydroxybut-2-yl)-pyrazine-2,5-dicarboxamide,
-N-méthyl-N-(1,3,4-trihydroxybut-2-yl)-N'-méthyl-N'-(1,3,4-trihydroxybut-2-yl)-pyrazine-2,6-dicarboxamide,
-N,N'-bis-[2-(hydroxyméthyl)-3-hydroxyprop-1-yl]-pyrazine-2,5-dicarboxamide,
-N,N'-bis-[2-(hydroxyméthyl)-3-hydroxyprop-1-yl]-pyrazine-2,6-dicarboxamide,
-N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,5-dicarboxamide,
-N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,6-dicarboxamide,
-N,N,N',N'-tétrakis-[2-(hydroxyméthyl)-3-hydroxyprop-1-yl]-pyrazine-2,5-dicarboxamide,
-N,N,N',N'-tétrakis-[2-(hydroxyméthyl)-3-hydroxyprop-1-yl]-pyrazine-2,6-dicarboxamide,
-N,N'-bis-[1,1,1-tri-(hydroxyméthyl)-méthyl]-pyrazine-2,5-dicarboxamide,
-N,N'-bis-[1,1,1-tri-(hydroxyméthyl)-méthyl]-pyrazine-2,6-dicarboxamide,
-N-méthyl-N-[1,1,1-tri-(hydroxyméthyl)-méthyl]-N'-méthyl-N'-[1,1,1-tri-(hydroxyméthyl)-méthyl]-pyrazine-2,5-dicarboxamide,
-N-méthyl-N-[1,1,1-tri-(hydroxyméthyl)-méthyl]-N'-méthyl-N'-[1,1,1-tri-(hyd roxyméthyl)-méthyl]-pyrazine-2,6-dicarboxamide,
-N,N'-bis-[1,1-di(hydroxyméthyl)-éth-1-yl]-pyrazine-2,5-dicarboxamide,
-N,N'-bis-[1,1-di(hydroxyméthyl)-éth-1yl]-pyrazine-2,6-dicarboxamide,
-N-méthyl-N-[1,1-di-(hydroxyméthyl)-éth-1-yl]-N'-méthyl-N'-[1,1-di-(hydroxyméthyl)-éth-1-yl]-pyrazine-2,5-dicarboxamide,
-N-méthyl-N-[1,1-di-(hydroxyméthyl)-éth-1-yl]-N'-méthyl-N'-[1,1-di-(hydroxyméthyl)-éth-1-yl]-pyrazine-2,6-dicarboxamide,
-N,N,N',N'-tétrakis-(1,3-dihydroxyprop-2-yl]-pyrazine-2,5-dicarboxamide,
-N,N,N',N'-tétrakis-(1,3-dihydroxyprop-2-yl]-pyrazine-2,6-dicarboxamide,
-N,N'-bis-(2-hydroxyéthyl)-pyrazine-2,5-diméthanamine,
-N,N'-bis-(2-hydroxyéthyl)-pyrazine-2,6- diméthanamine,
-N-méthyl-N-(2-hydroxyéthyl)-N'-méthyl-N'(2-hydroxyéthyl)-pyrazine-2,5-diméthanamine,
-N-méthyl-N-(2-hydroxyéthyl)-N'-méthyl-N'(2-hydroxyéthyl)-pyrazine-2,6-diméthanamine,
-N,N,N',N'-tétrakis(2-hydroxyéthyl)-pyrazine-2,5-diméthanamine,
-N,N,N',N'-tétrakis(2-hydroxyéthyl)-pyrazine-2,6-diméthanamine,
-N,N'-bis-(2,3-dihydroxyprop-1-yl)-pyrazine-2,5-diméthanamine,
-N,N'-bis-(2,3-dihydroxyprop-1-yl)-pyrazine-2,6-diméthanamine,
-N-méthyl-N-(2,3-dihydroxyprop-1-yl)-N'-méthyl-N'-(2,3-dihydroxyprop-1-yl)-pyrazine-2,5- diméthanamine,
-N-méthyl-N-(2,3-dihydroxyprop-1-yl)-N'-méthyl-N'-(2,3-dihydroxyprop-1-yl)-pyrazine-2,6- diméthanamine,
-N,N,N',N'-tétrakis-(2,3-dihydroxyprop-1-yl)-pyrazine-2,5-diméthanamine,
-N,N,N',N'-tétrakis-(2,3-dihydroxyprop-1-yl)-pyrazine-2,6- diméthanamine,
-N,N'-bis-(1,3-dihydroxyprop-2-yl)-pyrazine-2,5-diméthanamine,
-N,N'-bis-(1,3-dihydroxyprop-2-yl]-pyrazine-2,6-diméthanamine,
-N-méthyl-N-(1,3-dihydroxyprop-2-yl)-N'-méthyl-N'-(1,3-dihydroxyprop-2-yl)-pyrazine-2,5- diméthanamine,
-N-méthyl-N-(1,3-dihydroxyprop-2-yl)-N'-méthyl-N'-(1,3-dihydroxyprop-2-yl)-pyrazine-2,6- diméthanamine,
-N,N,N',N'-tétrakis-(1,3-dihydroxyprop-2-yl)-pyrazine-2,5-diméthanamine,
-N,N,N',N'-tétrakis-(1,3-dihydroxyprop-2-yl)-pyrazine-2,6-diméthanamine,
-N,N'-bis-(2,3,4-trihydroxybut-1-yl)-pyrazine-2,5-diméthanamine,
-N,N'-bis-(2,3,4-trihydroxybut-1-yl)-pyrazine-2,6- diméthanamine,
-N-méthyl-N-(2,3,4-trihydroxybut-1-yl)-N'-méthyl-N'-(2, 3,4-trihydroxyb ut-1-yl)-pyrazine-2,5- diméthanamine,
-N-méthyl-N-(2,3,4-trihydroxybut-1-yl)-N'-méthyl-N'-(2,3,4-trihydroxybut-1-yl)-pyrazine-2,6- diméthanamine,
-N,N'-bis-(1,3,4-trihydroxybut-2-yl)-pyrazine-2,5-diméthanamine,
-N,N'-bis-(1,3,4-trihydroxybut-2-yl)-pyrazine-2,6-diméthanamine,
-N-méthyl-N-(1,3,4-trihydroxybut-2-yl)-N'-méthyl-N'-(1,3,4-trihydroxybut-2-yl)-pyrazine-2,5-diméthanamine,
-N-méthyl-N-(1,3,4-trihydroxybut-2-yl)-N'-méthyl-N'-(1,3,4-trihydroxybut-2-yl)-pyrazine-2,6-diméthanamine,
-N,N'-bis-[2-(hydroxyméthyl)-3-hydroxyprop-1-yl]-pyrazine-2,5-diméthanamine,
-N,N'-bis-[2-(hydroxyméthyl)-3-hydroxyprop-1-yl]-pyrazine-2,6-diméthanamine,
-N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,5-diméthanamine,
-N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,6-diméthanamine,
-N,N,N',N'-tétrakis-[2-(hydroxyméthyl)-3-hydroxyprop-1-yl]-pyrazine-2,5-diméthanamine,
-N,N,N',N'-tétrakis-[2-(hydroxyméthyl)-3-hydroxyprop-1-yl]-pyrazine-2,6-diméthanamine,
-N,N'-bis-[1,1,1-tri-(hydroxyméthyl)-méthyl]-pyrazine-2,5-diméthanamine,
-N,N'-bis-[1,1,1-tri-(hydroxyméthyl)-méthyl]-pyrazine-2,6-diméthanamine,
-N-méthyl-N-[1,1,1-tri-(hydroxyméthyl)-méthyl]-N'-méthyl-N'-[1,1,1-tri-(hydroxyméthyl)-méthyl]-pyrazine-2,5-diméthanamine,
-N-méthyl-N-[1,1,1-tri-(hydroxyméthyl)-méthyl]-N'-méthyl-N'-[1,1,1-tri-(hydroxyméthyl)-méthyl]-pyrazine-2,6-diméthanamine,
-N,N'-bis-[1,1-di(hydroxyméthyl)-éth-1-yl]-pyrazine-2,5-diméthanamine,
-N,N'-bis-[1,1-di(hydroxyméthyl)-éth-1yl]-pyrazine-2,6-diméthanamine,
-N-méthyl-N-[1,1-di-(hydroxyméthyl)-éth-1-yl]-N'-méthyl-N'-[1,1-di-(hydroxyméthyl)-éth-1-yl]-pyrazine-2,5-diméthanamine,
-N-méthyl-N-[1,1-di-(hydroxyméthyl)-éth-1-yl]-N'-méthyl-N'-[1,1-di-(hydroxyméthyl)-éth-1-yl]-pyrazine-2,6-diméthanamine,
-2,5-bis-(2-hydroxyéthyloxyméthyl)-pyrazine,
-2,6-bis-(2-hydroxyéthyloxyméthyl)-pyrazine,
-2.5-bis-[(2R)-2,3-dihydroxyprop-1-yloxyméthyl]-pyrazine,
-2,5-bis-[(2S)-2,3-dihydroxyprop-1-yloxyméthyl]-pyrazine,
-2,6-bis-[(2R)-2,3-dihydroxyprop-1-yloxyméthyl]-pyrazine,
-2,6-bis-[(2S)-2,3-dihydroxyprop-1-yloxyméthyl]-pyrazine,-2,5-bis-(1,3-dihydroxyprop-2-yloxyméthyl)-pyrazine,
-2,6-bis-(1,3-dihydroxyprop-2-yloxyméthyl]-pyrazine,
-2,5-bis-(2,3,4-trihydroxybut-1-yloxyméthyl)-pyrazine,
-2,6-bis-(2,3,4-trihydroxybut-1-yloxyméthyl)-pyrazine,
-2,5-bis-(1,3,4-trihydroxybut-2-yloxyméthyl)-pyrazine,
-2,6-bis-(1,3,4-trihydroxybut-2-yloxyméthyl)-pyrazine,
-2,5-bis-[2-(hydroxyméthyl)-3-hydroxyprop-1-yloxyméthyl]-pyrazine,
-2,6-bis-[2-(hydroxyméthyl)-3-hydroxyprop-1-yloxyméthyl]-pyrazine,
-2,5-bis-[1,1,1-tri-(hydroxyméthyl)-méthyloxyméthyl]-pyrazine,
-2,6-bis-[1,1,1-tri-(hydroxyméthyl)- méthyloxyméthyl]-pyrazine,
-2,5-bis-[1,1-di(hydroxyméthyl)-éth-1-yloxyméthyl]-pyrazine,
-2,6-bis-[1,1-di(hydroxyméthyl)-éth-1yloxyméthyl]-pyrazine,
-bis-N,N'-(2-hydroxyéthylamido)-2,5-pyrazine,
-bis-N,N'-(2-hydroxyéthylamido)-2,6-pyrazine,
-bis-[N-méthyl-(2-hydroxyéthylamido)]-2,5-pyrazine,
-bis-[N-méthyl-(2-hydroxyéthylamido)]-2,6-pyrazine,
-bis-(2,3-dihydroxyprop-1-ylamido)-2,5-pyrazine,
-bis-(2,3-dihydroxyprop-1-ylamido)-2,6-pyrazine
-bis-[N-méthyl-(2,3-dihydroxyprop-1-ylamido)]-2,5-pyrazine,
-bis-[N-méthyl-(2,3-dihydroxyprop-1-ylamido)]-2,6-pyrazine,
-bis-(N-méthyl-((1,3-dihydroxyprop-2-ylamido)-2,5-pyrazine,
-bis-(N-méthyl-((1,3-dihydroxyprop-2-ylamido)-2,6-pyrazine,
-bis-(2,3,4-trihydroxybut-1-ylamido)-2,5-pyrazine,
-bis-(2,3,4-trihydroxybut-1-ylamido)-2,6-pyrazine,
-bis-[N-méthyl-(2,3,4-trihydroxybut-1-yl amido)]-2,5-pyrazine,
-bis-[N-méthyl-(2,3,4-trihydroxybut-1-yl amido)]-2,6-pyrazine,
-bis-(1,3,4-trihydroxybut-2-ylamido)-2,5-pyrazine,
-bis-(1,3,4-trihydroxybut-2-ylamido)-2,6-pyrazine,
-bis-[N-méthyl-(1,3,4-trihydroxybut-2-yl amido)]-2,5-pyrazine,
-bis-[N-méthyl-(1,3,4-trihydroxybut-2-yl amido)]-2,6-pyrazine,
-bis-[2-(hydroxyméthyl)-3-hydroxyprop-1-ylamido]-2,5-pyrazine,
-bis-[2-(hydroxyméthyl)-3-hydroxyprop-1-ylamido]-2,6-pyrazine,
-bis-{[N-méthyl-[2-(hydroxyméthyl)-3-hydroxyprop-1-ylamido]}-2,5-pyrazine
-bis-{[N-méthyl-[2-(hydroxyméthyl)-3-hydroxyprop-1-ylamido]}-2,6-pyrazine
-bis-[1,1,1-tri-(hydroxyméthyl)-méthylamido]-2,5-pyrazine,
-bis-[1,1,1-tri-(hydroxyméthyl)-méthylamido]-2,6-pyrazine,
-bis-{[N-méthyl-[1,1,1-tri-(hydroxyméthyl)-méthylylamido]}-2,5-pyrazine
-bis-{[N-méthyl-[1,1,1-tri-(hydroxyméthyl)-méthylylamido]}-2,6-pyrazine
-bis-[1,1-di(hydroxyméthyl)-éth-1-ylamido]-2,5-pyrazine,
-bis-[1,1-di(hydroxyméthyl)-éth-1-ylamido]-2,6-pyrazine,
-bis-{[N-méthyl-[1,1-di-(hydroxyméthyl)-éth-1-ylamido]}-2,5-pyrazine
-bis-{[N-méthyl-[1,1-di-(hydroxyméthyl)-éth-1-ylamido]}-2,6-pyrazine
ou un stéréoisomère de ces composés ou un sel d'un tel composé avec un acide minéral ou organique pharmaceutiquement acceptable.

Les médicaments particulièrement préférés sont ceux qui contiennent un composé de formule (I) dans laquelle
R₁ représente un radical -CO-NR₅R₆, -CO-N(CH₂-(CHOH)ₙ-CH₂OH)₂ dans lequel n est égal à 0, 1, 2, 3 ou 4, -CH₂-NR₅R₆, -CH₂-N(CH₂-(CHOH)_{y}-CH₂OH)₂ dans lequel y est égal à 0, 1, 2,
3 ou 4, et l'un des substituants R₃ ou R₄ représente un atome d'hydrogène et l'autre est identique à R₁, R₂ représente un atome d'hydrogène, R₅ représente un atome d'hydrogène ou un radical alkyle et R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃ ou,
alk représente un radical alkyle
leurs stéréoisomères et leurs sels avec un acide minéral ou organique pharmaceutiquement acceptable.

Les médicaments encore plus particulièrement préférés sont ceux qui contiennent en tant que principe actif un composé de formule (I) choisi parmi les composés suivants :
- N,N'-bis-[(tris-(hydroxyméthyl)méthyl]-pyrazine-2,5-dicarboxamide
- N-méthyl-N-[(2S,3R,4R,5R)-2,3,4,5-tétrahydroxyhex-1-yl]-N'-méthyl-N'-[(2S,3R,4R,5R)-2,3,4,5-tetrahydroxyhex-1-yl]-pyrazine-2,5-dicarboxamide
- N,N'-bis-(1,3-dihydroxyprop-2-yl)-pyrazine-2,5-dicarboxamide
- N,N'-bis-(2,3-dihydroxyprop-1-yl)-pyrazine-2,5-dicarboxamide
- N,N,N',N'-tétrakis(2-hydroxyéthyl)-pyrazine-2,5-dicarboxamide
- bis-{N-[2(R,S)-3-dihydroxyprop-1-yl]-N-méthyl}-pyrazine-2,5-dicarboxamide
- N,N'-bis-(2-méthoxyéthyl)-pyrazine-2,5-dicarboxamide
- N,N'-bis-(2-hydroxyéthyloxyéthyl)-pyrazine-2,5-dicarboxamide
- N,N'-bis(1,3-dihydroxyprop-2-yl)-pyrazine-2,5-diméthanamine
- N,N,N',N'-tétrakis(2-hydroxyéthyl)-pyrazine-2,5-diméthanamine
- N,N'-bis-(1,3-dihydroxyprop-2-yl)-pyrazine-2,5-diméthanamine
- N-méthyl-N-[(2S,3R,4R,5R)-2,3,4,5-tétrahydoxyhex-1-yl]-N'-méthyl-N'[(2S,3R,4R,5R)-2,3,4,5-tétrahydroxyhex-1-yl]-pyrazine-2,5-diméthanamine
ou un sel d'un tel composé avec un acide minéral ou organique pharmaceutiquement acceptable.

Les composés de formule (I) préférés sont ceux pour lesquels :
R₁ représente un radical -CO-NR₅R₆, -CO-N(CH₂-(CHOH)ₙ-CH₂OH)₂ dans lequel n est égal à 0, 1, 2, 3 ou 4, -CH₂-NR₅R₆, -CH₂-N(CH₂-(CHOH)_{y}-CH₂OH)₂ dans lequel y est égal à 0, 1, 2, 3 ou 4, et l'un des substituants R₃ ou R₄ représente un atome d'hydrogène et l'autre est identique à R₁, R₂ représente un atome d'hydrogène, R₅ représente un atome d'hydrogène ou un radical alkyle et R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃ ou,
alk représente un radical alkyle
leurs stéréoisomères et leurs sels avec un acide minéral ou organique pharmaceutiquement acceptable.

Les composés de formule (I) encore plus particulièrement préférés sont les composés suivants :
- N,N'-bis-[(tris-(hydroxyméthyl)méthyl]-pyrazine-2,5-dicarboxamide
- N-méthyl-N-[(2S,3R,4R,5R)-2,3,4,5-tétrahydroxyhex-1-yl]-N'-méthyl-N'-[(2S,3R,4R,5R)-2,3,4,5-tetrahydroxyhex-1-yl]-pyrazine-2,5-dicarboxamide
- N,N'-bis-(1,3-dihydroxyprop-2-yl)-pyrazine-2,5-dicarboxamide
- N,N'-bis-(2,3-dihydroxyprop-1-yl)-pyrazine-2,5-dicarboxamide
- N,N,N',N'-tétrakis(2-hydroxyéthyl)-pyrazine-2,5-dicarboxamide
- bis-{N-[2(R,S)-3-dihydroxyprop-1-yl]-N-méthyl}-pyrazine-2,5-dicarboxamide
- N,N'-bis-(2-méthoxyéthyl)-pyrazine-2,5-dicarboxamide
- N,N'-bis-(2-hydroxyéthyloxyéthyl)-pyrazine-2,5-dicarboxamide
- N,N'-bis(1,3-dihydroxyprop-2-yl)-pyrazine-2,5-diméthanamine
- N,N,N',N'-tétrakis(2-hydroxyéthyl)-pyrazine-2,5-diméthanamine
- N,N'-bis-(1,3-dihydroxyprop-2-yl)-pyrazine-2,5-diméthanamine
- N-méthyl-N-[(2S,3R,4R,5R)-2,3,4,5-tétrahydoxyhex-1-yl]-N'-méthyl-N'[(2S,3R,4R,5R)-2,3,4,5-tétrahydroxyhex-1-yl]-pyrazine-2,5-diméthanamine
et leurs sels avec un acide minéral ou organique.

Les composés de formule (I) pour lesquels R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, et l'un des substituants R₃ et R₄ représente un atome d'hydrogène et l'autre est identique à R₁ et R₂ représente un atome d'hydrogène peuvent être préparés par action d'un dérivé de formule : 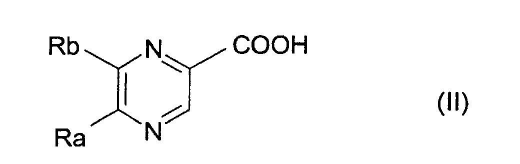 dans laquelle l'un des substituants Ra ou Rb représente un radical carboxy et l'autre un atome d'hydrogène, ou un dérivé réactif de ce diacide, sur une amine HNRcRd (III) dans laquelle soit Rc représente un atome d'hydrogène
ou un radical alkyle et Rd représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂ -CH₂-CH(CH₂OH)₂ ou -CH(CH₂OH)(CHOH)ₓ-CH₂OH dans lequel x est égal à 1, 2, 3 ou 4 et alk représente un radical alkyle, soit Rc et Rd sont identiques et représentent chacun un radical -CH₂-(CHOH)ₙ-CH₂OH dans lequel n est égal à 0, 1, 2, 3 ou 4, -CH(CH₂OH)₂, -CH₂OH ou -CH₂-CH(CH₂OH)₂.

Lorsque l'on met en oeuvre le diacide (II), on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2-éthane, chloroforme par exemple) à une température comprise entre 0°C et la température de reflux du mélange réactionnel. Lorsque l'on met en oeuvre un diester, on opère alors soit en milieu organique, éventuellement en présence d'un accepteur d'acide tel qu'une base organique azotée (trialkylamine, pyridine, diaza-1,8-bicyclo[5.4.0]undécène-7 ou diaza-1,5-bicyclo[4.3.0]nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les dérivés de formule (II) et leurs dérivés réactifs sont commercialisés ou peuvent être obtenus par application ou adaptation des méthodes décrites par W.J. Schut, H.I.X. Mager et W. Berends, Rec. Trav. Chim. Pays-Bas , 80, 391 (1961) et H.I.X. Mager et W. Berends, Rec. Trav. Chim. Pays-Bas, 77,827 (1958) ou par oxydation du dérivé diméthylé correspondant, par exemple au moyen d'oxyde de sélénium, au sein d'un mélange eau-pyridine, à une température comprise entre 60 et 100°C. Les diesters sont obtenus par estérification des diacides, de préférence au moyen d'un alcool aliphatique inférieur (1-4C), en présence d'un acide (de préférence l'acide sulfurique), à la température d'ébullition du milieu réactionnel.

Les dérivés diméthylés correspondants sont commerciaux ou peuvent être obtenus notamment par adaptation ou application des méthodes décrites dans «The Pyrazines» G.B. Barlin Chapitre 4, pp68-77 dans «The Chemistry of Heterocyclic Compounds» A. Weissberger et E.C. Taylor Editeurs- 1982-Interscience Publishers.

Les amines de formule (III) sont commercialisées ou peuvent être obtenues notamment par adaptation ou application des méthodes décrites dans Houben-Weyl «Methoden der Organischen Chemie» Volume XI/1 pp1-1037-Georg Thieme Verlag -Stuttgart (1957).

Les composés de formule (I) pour lesquels R₁ représente un radical -CH₂-O-R₆ et l'un des substituants R₃ ou R₄ représente un atome d'hydrogène et l'autre est identique à R₁ et R₂ représente un atome d'hydrogène peuvent être préparés par action d'un dérivé de formule : 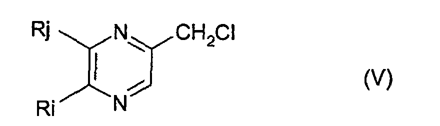 dans laquelle l'un des substituants Ri ou Rj représente un radical -CH₂Cl et l'autre un atome d'hydrogène sur un dérivé R₆OH (VI) pour lequel R₆ a les mêmes significations que dans la formule (I) et dans lequel les fonctions hydroxy sont éventuellement protégées sous forme d'acétal cyclique.

Cette réaction s'effectue généralement par action de l'alcoolate de métal alcalin (sodium ou potassium de préférence) correspondant à R₆OH dans un solvant inerte tel qu'un éther (tétrahydrofuranne, dioxanne par exemple), un amide (diméthylformamide), à une température comprise entre 0°C et la température d'ébullition du mélange réactionnel. L'alcoolate de métal alcalin peut être obtenu par action d'une base (hydroxyde ou méthylate de sodium ou de potassium) sur l'alcool R₆OH correspondant.

Les dérivés de formule (V) sont commercialisés ou peuvent être obtenus par les méthodes décrites dans «The Pyrazines» G.B. Barlin Chapitre 5, pp114-116 dans «The Chemistry of Heterocyclic Compounds» A. Weissberger et E.C. Taylor Editeurs - 1982 - Interscience Publishers, et notamment par halogénation du dérivé diméthylé correspondant au moyen d'un agent halogénant tel que la N-chlorosuccinimide dans un solvant inerte tel que le tétrachlorure de carbone, en présence d'un peroxyde d'alkyle (par exemple le peroxyde de benzoyle), à une température comprise entre 40°C et la température d'ébullition du milieu réactionnel.

Les dérivés R₆OH (VI) sont commercialisés ou peuvent être obtenus notamment par application ou adaptation des méthodes décrites dans Houben-Weyl «Methoden der Organischen Chemie» Volume VI/1A partie 1, pp1-653 - 4eme édition - Georg Thieme Verlag -Stuttgart (1979), volume VI/1A partie 2, pp771-1516 - 4eme édition - Georg Thieme Verlag -Stuttgart (1980) et volume VI/1B pp1-976 - 4eme édition - Georg Thieme Verlag-Stuttgart (1984).

Les composés de formule (I) pour lesquels R₁ représente un radical -CH₂-NR₅R₆, -CH₂-N(CH₂OH)₂, -CH₂-N(CH₂-(CHOH)_{y}-CH₂OH)₂, -CH₂-N(CH(CH₂OH)₂)₂ ou -CH₂-N(CH₂-CH(CH₂OH)₂)₂ et l'un des substituants R₃ ou R₄ représente un atome d'hydrogène et l'autre est identique à R₁, et R₂ représente un atome d'hydrogène, peuvent être préparés par action d'un dérivé de formule (V) dans laquelle l'un des substituants Ri et Rj représente un radical -CH₂Cl et l'autre un atome d'hydrogène sur une amine HNRcRd (III) dans laquelle Rc et Rd ont les mêmes significations que précédemment.

Cette réaction s'effectue généralement dans un solvant inerte tel qu'un éther (tétrahydrofuranne, dioxanne par exemple) ou un solvant chloré (tétrachlorure de carbone, chlorure de méthylène, dichloro-1,2 éthane, chloroforme par exemple) à une température comprise entre 0°C et la température d'ébullition du mélange réactionnel, éventuellement en présence d'un accepteur d'acide tel qu'une base organique azotée (trialkylamine, pyridine, diaza-1,8-bicyclo[5.4.0]undécène-7 ou diaza-1,5-bi-cyclo[4.3.0]nonène-5 par exemple).

Les composés de formule (I) pour lesquels R₁ représente un radical -NR₅-CO-R₆ et l'un des substituants R₃ ou R₄ représente un atome d'hydrogène et l'autre est identique à R₁, et R₂ représente un atome d'hydrogène peuvent être préparés par action d'un dérivé de formule : 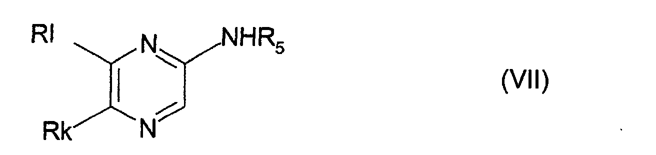 dans laquelle R₅ a les mêmes significations que dans la formule (I) et l'un des substituants Rk ou RI représente un atome d'hydrogène et l'autre un radical NHR₅ dans lequel R₅ a les mêmes significations que dans la formule (I) sur un acide HOOC-R₆ (VIII) pour lequel R₆ a les mêmes significations que dans la formule (I) ou un dérivé réactif de cet acide et, en particulier, un halogénure de cet acide.

Cette réaction s'effectue généralement en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel, en présence d'un accepteur d'acide tel qu'une base organique azotée (trialkylamine, pyridine, diaza-1,8-bicyclo[5.4.0]undécène-7 ou diaza-1,5-bi-cyclo[4.3.0]nonène-5 par exemple), dans un solvant tel que cité ci-dessus, ou un mélange de ces solvants, à une température comprise entre 0°C et la température d'ébullition du mélange réactionnel. Lorsque l'on utilise un chlorure de l'acide (VIII), on opère généralement au sein du tétrahydrofuranne, à une température comprise entre 0°C et la température de reflux du mélange réactionnel.

Les dérivés de formule (VII) sont commercialisés ou peuvent être obtenus notamment par application ou adaptation des méthodes décrites dans «The Pyrazines» G.B. Barlin Chapitre 8, pp205-246 dans « The Chemistry of Heterocyclic Compounds » A. Weissberger et E.C. Taylor Editeurs - 1982-Interscience Publishers,

Les dérivés HOOC-R₆ (VIII) sont commercialisés ou peuvent être obtenus notamment par application ou adaptation des méthodes décrites dans Houben-Weyl «Methoden der Organischen Chemie» Volume VIII Georg Thieme Verlag -Stuttgart (1952).

Les composés de formule (I) pour lesquels R₁ représente un radical -CH₂-O-R₆ dans lequel R₆ représente un radical -alk-O-alk et l'un des substituants R₃ ou R₄ représente un atome d'hydrogène et l'autre est identique à R₁ et R₂ représente un atome d'hydrogène peuvent être préparés par action d'un dérivé de formule : 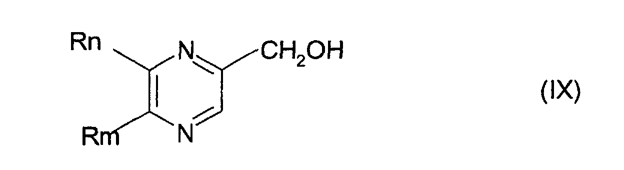 dans laquelle l'un des substituants Rm ou Rn représente un radical -CH₂OH et l'autre un atome d'hydrogène sur un dérivé Hal-alk-O-alk (X) pour lequel alk représente un radical alkyle et Hal représente un atome d'halogène et, de préférence, un atome de chlore ou de brome.

Cette réaction s'effectue généralement au sein d'un solvant inerte tel que le tétrahydrofuranne, à 20°C.

Les dérivés de formule (IX) sont commercialisés ou peuvent être obtenus par les méthodes décrites dans « The Pyrazines » G.B. Barlin dans « The Chemistry of Heterocyclic Compounds » A. Weissberger et E.C. Taylor Editeurs - 1982 - Interscience Publishers.

Les dérivés Hal-alk-O-alk sont commercialisés ou peuvent être obtenus selon les méthodes décrites dans Houben-Weyl «Methoden der Organischen Chemie» Volume V/4 Georg Thieme Verlag -Stuttgart (1960).

Les différents stéréoisomères des composés de formule (I) sont obtenus à partir des stéréoisomères correspondants des différents intermédiaires.

Il est entendu pour l'homme du métier que, pour la mise en oeuvre des procédés selon l'invention décrits précédemment, il peut être nécessaire d'introduire des groupes protecteurs des fonctions hydroxy afin d'éviter des réactions secondaires. Ces groupes sont ceux qui permettent d'être éliminés sans toucher au reste de la molécule. Comme exemples de groupes protecteurs de la fonction hydroxy, on peut citer les trialkylsilyle (triéthylsilyle par exemple), benzyle. D'autres groupes protecteurs utilisables dans ces procédés sont également décrits par W. GREENE et coll., Protective Groups in Organic Synthesis, second edition, 1991, Jonh Wiley & Sons et P.J. KOCIENSKI, Protecting groups, éditeur Thieme Verlag (1994).

Les mélanges réactionnels obtenus par les divers procédés décrits précédemment sont traités selon des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

Les exemples suivants illustrent l'invention :

### Exemple 1

Une solution de 392 mg de 2,5-diméthoxycarbonylpyrazine et de 485 mg de tris(hydroxyméthyl)aminométhane dans 3 cm³ d'éthanol est chauffée à une température voisine du reflux durant 90 minutes. Le mélange réactionnel est refroidi à une température de 20°C puis 10 cm³ d'éthanol sont ajoutés en 2 minutes. Le mélange réactionnel est agité à 20°C durant 1 heure. Le solide blanc formé est filtré, lavé successivement par 2 fois 10 cm³ d'éthanol bouillant et 2 fois 10 cm³ d'éther de diéthyle. On obtient ainsi 650 mg de N,N'-bis-[(tris-(hydroxyméthyl)méthyl]-pyrazine-2,5-dicarboxamide sous la forme d'un solide blanc fondant à une température supérieure à 260°C [Spectre de R.M.N. ¹H (250 MHz, (CD₃)₂SO d6, δ en ppm) : 3,76 (d, J = 5,5 Hz, 12H : les 6 OCH₂); 4,90 (t, J = 5,5 Hz, 6H : les 6 OH); 8,25 (s, 2H : =CH en 3 et =CH en 6); 9,26 (s, 2H : les 2 CONH)].

La 2,5-diméthoxycarbonylpyrazine peut être obtenue selon W.J. Schut, H.I.X. Mager et W. Berends, Rec. Trav. Chim. Pays-Bas, 80, 391 (1961).

### Exemple 2

Une solution de 392 mg de 2,5-diméthoxycarbonylpyrazine et de 780 mg de N-méthylglucamine dans 5 cm³ d'éthanol est chauffée à une température voisine du reflux durant 3 heures et le mélange réactionnel est filtré à chaud. Le solide blanc est lavé par 2 fois 10 cm³ d'éthanol bouillant. On obtient ainsi 1000 mg de N-méthyl-N-[(2S,3R,4R,5R)-2,3,4,5-tétrahydroxyhex-1-yl]-N'-méthyl-N'-[(2S,3R,4R,5R)-2,3,4,5-tetrahydroxyhex-1-yl]-pyrazine-2,5-dicarboxamide sous la forme d'un solide blanc fondant à 152°C [Spectre de R.M.N. ¹H (400 MHz, (CD₃)₂SO d6, à une température de 403 K, δ en ppm) : 3,14 (s, 6H : les 2 NCH₃); de 3,45 à 4,28 (mts, 16H : les 8 CHO - les 2 CH₂O et les 2 CH₂N ); 8,78 (s, 2H : =CH en 3 et =CH en 6)].

### Exemple 3

Une solution de 392 mg de 2,5-diméthoxycarbonylpyrazine et de 364 mg de 2-amino-1,3-propanediol dans 5 cm³ d'éthanol est chauffée à une température voisine du reflux durant 3 heures. Le mélange réactionnel est filtré à chaud. Le solide blanc est lavé par 3 fois 10 cm³ d'éthanol bouillant. On obtient ainsi 530 mg de N,N'-bis-(1,3-dihydroxyprop-2-yl)-pyrazine-2,5-dicarboxamide sous la forme d'un solide blanc fondant à 218°C [Spectre de R.M.N. ¹H (400 MHz, (CD₃)₂SO d6, δ en ppm) : de 3,45 à 3,65 (mt, 8H : les 4 CH₂O); 4,02 (mt, 2H : les 2 NCH); 4,85 (t, J = 5,5 Hz, 4H : les 4 OH); 8,48 (d, J = 9 Hz, 2H : les 2 CONH); 9,25 (s, 2H : =CH en 3 et =CH en 6)].

### Exemple 4

Une solution de 490 mg de 2,5-diméthoxycarbonylpyrazine et de 0,40 cm³ de 3-amino-1,2-propanediol dans 5 cm³ d'éthanol est chauffée à une température voisine du reflux durant 3 heures. Le mélange réactionnel est filtré à chaud. Le solide blanc est lavé par 3 fois 15 cm³ d'éthanol bouillant. On obtient ainsi 416 mg de N,N'-bis-(2,3-dihydroxyprop-1-yl)-pyrazine-2,5-dicarboxamide sous la forme d'un solide blanc fondant à 234°C [Spectre de R.M.N. ¹H (400 MHz, (CD₃)₂SO d6, δ en ppm) : de 3,20 à 3,60 (mt, 8H : les 2 OCH₂ et les 2 NCH₂); 3,68 (mt, 2H : les 2 OCH); 4,67 (t, J = 5,5 Hz, 2H : OH en 2γ et OH en 5γ); 4,95 (d, J = 5 Hz, 2H : OH en 2β et OH en 5β); 8,79 (t, J = 5,5 Hz, 2H : les 2 CONH); 9,23 (s, 2H : =CH en 3 et =CH en 6)].

### Exemple 5

Une solution de 500 mg de 2,5-diméthoxycarbonylpyrazine et de 0,48 cm³ de diéthanolamine dans 3 cm³ d'éthanol est chauffée à une température voisine du reflux durant 3 heures. Le mélange réactionnel est refroidi à 20°C puis le solide blanc formé est filtré et lavé par 2 cm³ d'éthanol. On obtient ainsi 600 mg de N,N,N',N'-tétrakis(2-hydroxyéthyl)-pyrazine-2,5-dicarboxamide sous la forme d'un solide blanc fondant à 143°C [Spectre de R.M.N. ¹H (250 MHz, (CD₃)₂SO d6, à une température de 403 K, δ en ppm) : de 3,50 à 3,80 (mt, 16H : les 4 NCH₂CH₂O); 4,32 (mt, 4H : les 4 OH); 8,76 (s, 2H : =CH en 3 et =CH en 6)].

### Exemple 6

Une solution de 500 mg de 2,5-diméthoxycarbonylpyrazine et de 525 mg de 2-amino-2-méthyl-1,3-propanediol dans 3 cm³ d'éthanol est chauffée à une température voisine du reflux durant 90 minutes. Le mélange réactionnel est filtré à chaud. Le solide blanc est lavé par 5 cm³ d'éthanol bouillant. On obtient ainsi 200 mg de N,N'-bis-(1,3-dihydroxy-2-méthylprop-2-yl)-pyrazine-2,5-dicarboxamide sous la forme d'un solide blanc fondant à 208°C [Spectre de R.M.N. ¹H (400 MHz, CDCI3, δ en ppm): 1,34 (s, 6H : les 2 CH₃); 3,55 et 3,66 (2 dd, J = 11 et 5,5 Hz, 8H : les 4 OCH2); 5,01 (t, J = 5,5 Hz, 4H : les 4 OH); 8,22 (s, 2H : =CH en 3 et =CH en 6); 9,20 (s, 2H : les 2 CONH)].

### Exemple 7

Une solution de 500 mg de 2,5-diméthoxycarbonylpyrazine et de 525 mg de 3-méthylamino-1,2-propanediol dans 3 cm³ d'éthanol est chauffée à une température voisine du reflux durant 2 heures. Le mélange réactionnel est refroidi à 20°C puis la solution obtenue est concentrée sous pression réduite (2,7 kPa) à 40°C. Le résidu est repris par 5 cm³ d'éthanol. La suspension est portée au reflux et filtrée à chaud. Après refroidissement du filtrat, le solide blanc formé est filtré puis lavé par 2 fois 10 cm³ d'éthanol. On obtient ainsi 230 mg de bis-{N-[2(R,S)-3-dihydroxyprop-1-yl]-N-méthyl}-pyrazine-2,5-dicarboxamide sous la forme d'un solide blanc fondant à 158°C [Spectre de R.M.N. ¹H (400 MHz, (CD₃)₂SO d6, à une température de 393 K, δ en ppm) : 3,12 (s, 6H : les 2 CONCH₃); de 3,30 à 3,65 (mf, 8H : NCH₂ en 2α - NCH₂ en 5α - OCH₂ en 2γ et OCH₂ 5γ); 3,82 (mf, 2H : CH 2β et CH 5β); 3,98 et 4,29 (2 mfs, 2H chacun : les 4 OH); 8,77 (s, 2H : =CH en 3 et =CH en 6)].

### Exemple 8

Une solution de 500 mg de 2,5-diméthoxycarbonylpyrazine et de 0,430 cm³ de 2-méthoxyéthylamine dans 3 cm³ d'éthanol est chauffée à une température voisine du reflux durant 5 heures. Le mélange réactionnel est refroidi à une température de 20°C puis le solide blanc formé est filtré et lavé par 2 fois 5 cm³ d'éthanol. On obtient ainsi 150 mg de N,N'-bis-(2-méthoxyéthyl)-pyrazine-2,5-dicarboxamide sous la forme d'un solide blanc fondant à 165°C [Spectre de R.M.N. ¹H (300 MHz, (CD₃)₂SO d6, δ en ppm) : 3,29 (s, 6H : les 2 OCH₃); 3,51 (mt, 8H : les 2 NCH₂CH₂O); 8,99 (mt, 2H : les 2 CONH); 9,22 (s, 2H : =CH en 3 et =CH en 6)].

### Exemple 9

Une solution de 500 mg de 2,5-diméthoxycarbonylpyrazine et de 0,720 cm³ de 2-(2-aminoéthoxy)éthanol dans 3 cm³ d'éthanol est chauffée à une température voisine du reflux durant 4 heures. Le mélange réactionnel est refroidi à une température de 20°C puis le solide blanc formé est filtré et lavé par 2 fois 5 cm³ d'éthanol. On obtient ainsi 150 mg de N,N'-bis-(2-hydroxyéthyloxyéthyl)-pyrazine-2,5-dicarboxamide sous la forme d'un solide blanc fondant à 125°C [Spectre de R.M.N. ¹H (300 Mhz, (CD₃)₂SO d6, δ en ppm) : de 3,35 à 3,65 (mt, 16H : les 2 NCH₂CH₂OCH₂CH₂O); 4,62 (t, J = 5,5 Hz, 2H : les 2 OH); 9,00 (t, J = 6 Hz, 2H : les 2 NHCO); 9,20 (s, 2H : =CH en 3 et =CH en 6)].

### Exemple 10

Une solution de 500 mg de 2,6-diméthoxycarbonylpyrazine et de 525 mg de 2-amino-2-méthyl-1,3-propanediol dans 5 cm³ d'éthanol est chauffée à une température voisine du reflux durant 3 heures. Le mélange réactionnel est refroidi à une température de 20°C puis le solide blanc formé est filtré et lavé par 2 fois 5 cm³ d'éthanol. On obtient ainsi 260 mg de N,N'-bis-(1,3-dihydroxy-2-méthylprop-2-yl)-pyrazine-2,6-dicarboxamide sous la forme d'un solide blanc fondant à 169°C [Spectre de R.M.N. ¹H (400 MHz, (CD₃)₂SO d6, δ en ppm) : 1,34 (s, 6H : les 2 CH₃); 3,61 et 3,69 (2 dd, J = 11 et 5,5 Hz, 8H : les 4 CH₂O); 4,88 (t, J = 5,5 Hz, 4H : les 4 OH); 8,16 (s, 2H : les 2 CONH); 9,35 (s, 2H : =CH en 3 et =CH en 5)].

La 2,6-diméthoxycarbonylpyrazine peut être obtenue comme décrit par H.I.X. Mager et W. Berends, Rec. Trav. Chim. Pays-Bas, 77,827 (1958).

### Exemple 11

A une solution de 1,80 g de 2,5-diméthylpyrazine dans 50 cm³ de tétrachlorure de carbone, on ajoute 110 mg de peroxyde de benzoyle et 4,43 g de N-chlorosuccinimide. Le mélange réactionnel est chauffé durant 24 heures à une température voisine du reflux. Le mélange réactionnel est refroidi à 20°C puis filtré. A la solution obtenue on ajoute une solution de 3,42 g de 2-amino-1,3-propanol dans 60 cm³ de tétrachlorure de carbone. Le mélange réactionnel est chauffé à une température voisine du reflux durant 4 heures puis concentré sous pression réduite (2,7 kPa) à 40°C. Le résidu huileux (7,0 g) est chromatographié sur une colonne de silice (0,02-0,045 mm), de 3,5 cm de diamètre, éluée avec un mélange chloroforme-méthanol-ammoniaque à 20 % (24:6:1 en volumes) en recueillant des fractions de 100 cm^{3,} Les fractions contenant le produit sont concentrées sous pression réduite (2,7 kPa) à 40°C. L'huile orange obtenue (1,0 g) est reprise par 15 cm³ d'éthanol bouillant. Le mélange est traité par 500 mg de noir animal et filtré à chaud. Le filtrat est abandonnée 2 heures à 20 °C. Le solide formé est filtré, rincé par 2 fois 2 cm³ d'éthanol puis séché sous pression réduite (2,7 kPa) à 40°C. On obtient ainsi 260 mg de N,N'-bis(1,3-dihydroxyprop-2-yl)-pyrazine-2,5-diméthanamine sous la forme d'un solide blanc fondant à 132°C [Spectre de R.M.N. ¹H (300 MHz, (CD₃)₂SO d6, δ en ppm) : 2,56 (mt, 2H : les 2 NCH); de 3,20 à 3,50 (mt, 8H : les 4 OCH₂); 3,90 (s, 4H : les 2 ArCH₂N); 4,48 (t, J = 5 Hz, 4H : les 4 OH); 8,62 (s, 2H : =CH en 3 et =CH en 6)].

### Exemple 12

A une solution de 3,60 g de 2,5-diméthylpyrazine dans 100 cm³ de tétrachlorure de carbone, on ajoute 220 mg de peroxyde de benzoyle et 8,86 g de N-chlorosuccinimide. Le mélange réactionnel est chauffé durant 24 heures à une température voisine du reflux. Le mélange réactionnel est refroidi à 20°C puis filtré. A la solution obtenue on ajoute une solution de 24,11 cm³ de diéthanolamine dans 130 cm³ de tétrachlorure de carbone. Le mélange réactionnel est chauffé à une température voisine du reflux durant 4 heures. puis concentré sous pression réduite (2,7 kPa) à 40°C. Le résidu huileux est chromatographié sur une colonne de silice (0,02-0,045 mm), de 3,5 cm de diamètre, éluée avec un mélange chloroforme-méthanol-ammoniaque à 20 % (24:6:1 en volumes) en recueillant des fractions de 200 cm³. Les fractions contenant le produit sont concentrées sous pression réduite (2,7 kPa) à 40°C. L'huile brune obtenue (1,0 g) est reprise par 30 cm³ d'éthanol et 5 cm³ d'une solution d'éther chlorhydrique 2N puis 5 cm³ de diéthyléther. Le mélange est laissé 1 heure à 5°C. Le solide formé est filtré et rincé par 2 fois 10 cm³ de diéthyléther. Le solide est recristallisé dans 30 cm³ d'éthanol. On obtient ainsi 400 mg de N,N,N',N'-tétrakis(2-hydroxyéthyl)-pyrazine-2,5-diméthanamine sous la forme d'un solide blanc fondant à 185°C [Spectre de R.M.N. ¹H (400 MHz, (CD₃)₂SO d6 avec ajout de quelques gouttes de CD₃COOD d4, δ en ppm) : 3,39 et 3,85 (respectivement t large et t, J = 5 Hz, 8H chacun : les 4 NCH₂CH₂O); 4,74 (s, 4H : les 2 ArCH₂N); 8,97 (s, 2H : =CH en 3 et =CH en 6). Pour le spectre en (CD₃)₂SO d6, nous observons en plus : de 5,20 à 5,60 (mf étalé, 4H : les 4 OH); de 10,30 à 10,50 (mf, 2H : les 2 NH⁺Cl⁻)].

### Exemple 13

A une solution de 2,76 g de 2,6-diméthylpyrazine dans 60 cm³ de tétrachlorure de carbone, on ajoute 50 mg de peroxyde de benzoyle et 6,81 g de N-chlorosuccinimide. Le mélange réactionnel est chauffé durant 24 heures à une température voisine du reflux. Le mélange réactionnel est refroidi à 20°C puis filtré. A la solution obtenue on ajoute 100 cm³ de tétrachlorure de carbone puis une solution de 9,30 g de 2-amino-1,3-propanol dans 50 cm³ de tétrachlorure de carbone. Le mélange réactionnel est chauffé à une température voisine du reflux durant 6 h. puis concentré sous pression réduite (2,7 kPa) à 40°C. Le résidu huileux (13 g) est chromatographié sur une colonne de silice (0,02-0,045 mm), de 5 cm de diamètre, éluée avec un mélange chloroforme-méthanol-ammoniaque à 20 % (24:6:1 en volumes) pour les soixante premières fractions et un mélange chloroforme-méthanol-ammoniaque à 20 % (12:6:1 en volumes) pour les suivantes, en recueillant des fractions de 100 cm³. Les fractions contenant le produit (fractions 61 à 75) sont concentrées sous pression réduite (2,7 kPa) à 40°C. L'huile orange obtenue (4,7 g) est chromatographié sur une colonne de silice (0,02-0,045 mm), de 4 cm de diamètre, éluée avec un mélange chloroforme-méthanol-ammoniaque à 20 % (12:6:1 en volumes).en recueillant des fractions de 100 cm³. Les fractions contenant le produit sont concentrées sous pression réduite (2,7 kPa) à 40°C. Le résidu est trituré successivement avec 10 cm³ d'acétone et d'oxyde de diéthyle. Le solide résiduel est dissous dans 10 cm³ d'éthanol. A la solution obtenue, on ajoute goutte à goutte 30 cm³ d'oxyde d'isopropyle puis le mélange est laissé 48 heures à 20°C. Le solide orange formé est filtré, rincé par 3 fois 5 cm³ d'un mélange méthanol-oxyde d'isopropyle (1:9 en volumes) puis séché sous pression réduite (2,7 kPa) à 40°C. On obtient ainsi 260 mg de N,N'-bis-(1,3-dihydroxyprop-2-yl)-pyrazine-2,5-diméthanamine sous la forme d'un solide orange fondant à 87°C [Spectre de R.M.N. ¹H (400 MHz, (CD₃)₂SO d6, δ en ppm) : 2,58 (mt, 2H : les 2 NCH); de 3,30 à 3,50 (mt, 8H : les 4 CH₂O); 3,92 (s, 4H : les 2 ArCH₂N); 4,48 (mf, 4H : les 4 OH); 8,58 (s, 2H : =CH en 3 et =CH en 5)].

### Exemple 14

A une solution de 5,40 g de 2,5-diméthylpyrazine dans 150 cm³ de tétrachlorure de carbone, on ajoute 0,33 g de peroxyde de benzoyle et 13,30 g de N-chlorosuccinimide. Le mélange réactionnel est chauffé durant 24 heures à une température voisine du reflux puis le mélange réactionnel est refroidi à 20°C et filtré. A la solution obtenue on ajoute une solution de 7.32 g de N-méthylglucamine dans 60 cm³ de tétrachlorure de carbone. Le mélange réactionnel est chauffé à une température voisine du reflux durant 4 heures puis concentré sous pression réduite (2,7 kPa) à 40°C. L'huile résiduelle (10 g) est purifiée par chromatographie sur une colonne de silice (0,020-0,045 mm) en éluant avec un mélange chloroforme-méthanol-ammoniaque à 20% (24:6:1 en volumes) pour les 13 premières fractions et chloroforme-méthanol-ammoniaque à 20 % (12:6:2 en volumes) pour les suivantes, en récoltant des fractions de 100 cm³. Les fractions contenant le produit attendu sont réunies et concentrées sous pression réduite (2,7 kPa) à 50°C. L'huile jaune obtenue (1,3 g) est à nouveau purifiée par chromatographie sur une colonne de silice (0,020-0,045 mm) en éluant avec un mélange chloroforme-méthanol-ammoniaque à 20% (24:6:1 en volumes), en récoltant des fractions de 100 cm³. Les fractions contenant le produit attendu sont réunies et concentrées sous pression réduite (2,7 kPa) à 50°C. L'huile jaune obtenue (0.6 g) est cristallisée dans un mélange méthanol-méthylethylcétone-acétone. Le solide est filtré puis séché à poids constant pour conduire à 0,10 g de N-méthyl-N-[(2S,3R,4R,5R)-2,3,4,5-tétrahydoxyhex-1-yl]-N'-méthyl-N'[(2S,3R,4R,5R)-2,3,4,5-tétrahydroxyhex-1-yl]-pyrazine-2,5-diméthanamine sous forme d'un solide beige [Spectre de R.M.N. ¹H (400 MHz, (CD₃)₂SO d6 avec ajout de quelques gouttes de CD3COOD d4, δ en ppm) : 2,42 (s large, 6H : NCH₃); 2,72 et 2,83 (2 dd, respectivement J = 13 et 8 Hz et J = 13 et 4 Hz, 2H chacun : NCH₂); 3,40 et 3,59 (2 dd, respectivement J = 11 et 5,5 Hz et J = 11 et 2,5 Hz, 2H chacun : OCH₂ en κ); de 3,40 à 3,55 (mt, 4H : CH en ζ et CH en η); 3,67 (d, J = 2,5 Hz, 2H : CH en ε); 3,88 (mt, 2H : CH en d); 3,99 (AB limite, 4H : ArCH₂N); 8,71 (s, 2H : =CH).

Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant :

Des souris Swiss albinos pesant entre 22 et 26 g sont laissées à jeun pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.

Les souris ainsi choisies sont réparties en groupes d'au moins 10 animaux. Des groupes distincts reçoivent une solution de 3 à 50 mg/kg du produit à tester dans un véhicule tel que l'eau ou un mélange de méthylcellulose/tween et eau ou du véhicule une fois par jour par tubage gastrique. Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcéres des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoproteines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

L'invention concerne également l'utilisation des composés de formule générale (I) pour la préparation de compositions pharmaceutiques utiles pour le traitement ou la prévention du diabète et les complications du diabète.

## Revendications

1. Médicaments contenant en tant que principe actif au moins un composé de formule : 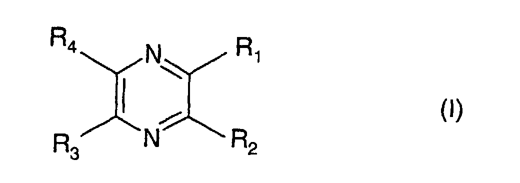 dans laquelle
R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ dans lequel n est égal à 0, 1, 2, 3 ou 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ dans lequel y est égal à 0, 1, 2, 3 ou 4, -CH₂-N(CH(CH₂OH)₂)₂ ou -CH₂-N(CH₂-CH(CH₂OH)₂)₂ et l'un des substituants R₃ ou R₄ représente un atome d'hydrogène et l'autre est identique à R₁ et R₂ représente un atome d'hydrogène,
R₅ représente un atome d'hydrogène ou un radical alkyle,
R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ ou -CH(CH₂OH)-(CHOH)ₓ-CH₂OH dans lequel x est égal à 1, 2, 3 ou 4,
alk représente un radical alkyle,
les radicaux alkyle contenant, sauf mention contraire, 1 à 6 atomes de carbone en chaîne droite ou ramifiée, leurs stéréoisomères et leurs sels avec un acide minéral ou organique.

2. Médicaments contenant un composé de formule (I) selon la revendication 1 choisi parmi les composés suivants :
- N,N'-bis-(2-hydroxyéthyl)-pyrazine-2,5-dicarboxamide,
- N,N'-bis-(2-hydroxyéthyl)-pyrazine-2,6-dicarboxamide,
- N-méthyl-N-(2-hydroxyéthyl)-N'-méthyl-N'(2-hydroxyéthyl)-pyrazine-2,5-dicarboxamide,
- N-méthyl-N-(2-hydroxyéthyl)-N'-méthyl-N'(2-hydroxyéthyl)-pyrazine-2,6-dicarboxamide,
- N,N,N',N'-tétrakis(2-hydroxyéthyl)-pyrazine-2,5-dicarboxamide,
- N,N,N',N'-tétrakis(2-hydroxyéthyl)-pyrazine-2,6-dicarboxamide,
- N,N'-bis-(2,3-dihydroxyprop-1-yl)-pyrazine-2,5-dicarboxamide,
- N,N'-bis-(2,3-dihydroxyprop-1-yl]-pyrazine-2,6-dicarboxamide,
- N-méthyl-N-(2,3-dihydroxyprop-1-yl)-N'-méthyl-N'-(2,3-dihydroxyprop-1-yl)-pyrazine-2,5-dicarboxamide,
- N-méthyl-N-(2,3-dihydroxyprop-1-yl)-N'-méthyl-N'-(-2,3-dihydroxyprop-1 -yl)-pyrazine-2,6-dicarboxamide,
- N,N,N',N'-tétrakis-(2,3-dihydroxyprop-1-yl)-pyrazine-2,5-dicarboxamide,
- N,N,N',N'-tétrakis-(2,3-dihydroxyprop-1-yl]-pyrazine-2,6-dicarboxamide,
- N,N'-bis-(1,3-dihydroxyprop-2-yl)-pyrazine-2,5-dicarboxamide,
- N,N'-bis-(1,3-dihydroxyprop-2-yl]-pyrazine-2,6-dicarboxamide,
- N-méthyl-N-(1,3-dihydroxyprop-2-yl)-N'-méthyl-N'-(1,3-dihydroxyprop-2-yl)-pyrazine-2,5-dicarboxamide,
- N-méthyl-N-(1,3-dihydroxyprop-2-yl)-N'-méthyl-N'-(1,3-dihydroxyprop-2-yl)-pyrazine-2,6-dicarboxamide,
- N,N,N',N'-tétrakis-(1,3-dihydroxyprop-2-yl)-pyrazine-2,5-dicarboxamide,
- N,N,N',N'-tétrakis-(1,3-dihydroxyprop-2-yl)-pyrazine-2,6-dicarboxamide,
- N,N'-bis-(2,3,4-trihydroxybut-1-yl)-pyrazine-2,5-dicarboxamide,
- N,N'-bis-(2,3,4-trihydroxybut-1-yl)-pyrazine-2,6-dicarboxamide,
- N-méthyl-N-(2,3,4-trihydroxybut-1-yl)-N'-méthyl-N'-(2, 3,4-trihydroxybut-1-yl)-pyrazine-2,5-dicarboxamide,
- N-méthyl-N-(2,3,4-trihydroxybut-1-yl)-N'-méthyl-N'-(2,3,4-trihydroxybut-1-yl)-pyrazine-2,6-dicarboxamide,
- N,N'-bis-(1,3,4-trihydroxybut-2-yl)-pyrazine-2,5-dicarboxamide,
- N,N'-bis-(1,3,4-trihydroxybut-2-yl)-pyrazine-2,6-dicarboxamide,
- N-méthyl-N-(1,3,4-trihydroxybut-2-yl)-N'-méthyl-N'-(1,3,4-trihydroxybut-2-yl)-pyrazine-2,5-dicarboxamide,
- N-méthyl-N-(1,3,4-trihydroxybut-2-yl)-N'-méthyl-N'-(1,3,4-trihydroxybut-2-yl)-pyrazine-2, 6-dicarboxamide,
- N,N'-bis-[2-(hydroxyméthyl)-3-hydroxyprop-1-yl]-pyrazine-2,5-dicarboxamide,
- N,N'-bis-[2-(hydroxyméthyl)-3-hydroxyprop-1-yl]-pyrazine-2,6-dicarboxamide,
- N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,5-dicarboxamide,
- N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,6-dicarboxamide,
- N,N,N',N'-tétrakis-[2-(hydroxyméthyl)-3-hydroxyprop-1-yl]-pyrazine-2,5-dicarboxamide,
- N,N,N',N'-tétrakis-[2-(hydroxyméthyl)-3-hydroxyprop-1-yl]-pyrazine-2,6-dicarboxamide,
- N,N'-bis-[1,1,1-tri-(hydroxyméthyl)-méthyl]-pyrazine-2,5-dicarboxamide,
- N,N'-bis-[1,1,1-tri-(hydroxyméthyl)-méthyl]-pyrazine-2,6-dicarboxamide,
- N-méthyl-N-[1,1,1-tri-(hydroxyméthyl)-méthyl]-N'-méthyl-N'-[1,1,1-tri-(hydroxyméthyl)-méthyl]-pyrazine-2,5-dicarboxamide,
- N-méthyl-N-[1,1,1-tri-(hydroxyméthyl)-méthyl]-N'-méthyl-N'-[1,1,1-tri-(hydroxyméthyl)-méthyl]-pyrazine-2,6-dicarboxamide,
- N,N'-bis-[1,1-di(hydroxyméthyl)-éth-1-yl]-pyrazine-2,5-dicarboxamide,
- N,N'-bis-[1,1-di(hydroxyméthyl)-éth-1yl]-pyrazine-2,6-dicarboxamide,
- N-méthyl-N-[1,1-di-(hydroxyméthyl)-éth-1-yl]-N'-méthyl-N'-[1,1-di-(hydroxyméthyl)-éth-1-yl]-pyrazine-2,5-dicarboxamide,
- N-méthyl-N-[1,1-di-(hydroxyméthyl)-éth-1-yl]-N'-méthyl-N'-[1,1-di-(hydroxyméthyl)-éth-1-yl]-pyrazine-2,6-dicarboxamide,
- N,N,N',N'-tétrakis-(1,3-dihydroxyprop-2-yl]-pyrazine-2,5-dicarboxamide,
- N,N,N',N'-tétrakis-(1,3-dihydroxyprop-2-yl]-pyrazine-2,6-dicarboxamide,
- N,N'-bis-(2-hydroxyéthyl)-pyrazine-2,5-diméthanamine,
- N,N'-bis-(2-hydroxyéthyl)-pyrazine-2,6-diméthanamine,
- N-méthyl-N-(2-hydroxyéthyl)-N'-méthyl-N'(2-hydroxyéthyl)-pyrazine-2,5-diméthanamine,
- N-méthyl-N-(2-hydroxyéthyl)-N'-méthyl-N'(2-hydroxyéthyl)-pyrazine-2,6-diméthanamine,
- N,N,N',N'-tétrakis(2-hydroxyéthyl)-pyrazine-2,5- diméthanamine,
- N,N,N',N'-tétrakis(2-hydroxyéthyl)-pyrazine-2,6- diméthanamine,
- N,N'-bis-(2,3-dihydroxyprop-1-yl)-pyrazine-2,5- diméthanamine,
- N,N'-bis-(2,3-dihydroxyprop-1-yl)-pyrazine-2,6- diméthanamine,
- N-méthyl-N-(2,3-dihydroxyprop-1-yl)-N'-méthyl-N'-(2,3-dihydroxyprop-1-yl)-pyrazine-2,5- diméthanamine,
- N-méthyl-N-(2,3-dihydroxyprop-1-yl)-N'-méthyl-N'-(2,3-dihydroxyprop-1-yl)-pyrazine-2,6- diméthanamine,
- N,N,N',N'-tétrakis-(2,3-dihydroxyprop-1-yl)-pyrazine-2,5-diméthanamine,
- N,N,N',N'-tétrakis-(2,3-dihydroxyprop-1-yl)-pyrazine-2,6-diméthanamine,
- N,N'-bis-(1,3-dihydroxyprop-2-yl)-pyrazine-2,5-diméthanamine,
- N,N'-bis-(1,3-dihydroxyprop-2-yl]-pyrazine-2,6-diméthanamine,
- N-méthyl-N-(1,3-dihydroxyprop-2-yl)-N'-méthyl-N'-(1,3-dihydroxyprop-2-yl)-pyrazine-2,5- diméthanamine,
- N-méthyl-N-(1,3-dihydroxyprop-2-yl)-N'-méthyl-N'-(1,3-dihydroxyprop-2-yl)-pyrazine-2,6- diméthanamine,
- N,N,N',N'-tétrakis-(1,3-dihydroxyprop-2-yl)-pyrazine-2,5-diméthanamine,
- N,N,N',N'-tétrakis-(1,3-dihydroxyprop-2-yl)-pyrazine-2,6-diméthanamine,
- N,N'-bis-(2,3,4-trihydroxybut-1-yl)-pyrazine-2,5-diméthanamine,
- N,N'-bis-(2,3,4-trihydroxybut-1-yl)-pyrazine-2,6-diméthanamine,
- N-méthyl-N-(2,3,4-trihydroxybut-1-yl)-N'-méthyl-N'-(2,3,4-trihydroxybut-1-yl)-pyrazine-2,5- diméthanamine,
- N-méthyl-N-(2,3,4-trihydroxybut-1-yl)-N'-méthyl-N'-(2,3,4-trihydroxybut-1-yl)-pyrazine-2,6- diméthanamine,
- N,N'-bis-(1,3,4-trihydroxybut-2-yl)-pyrazine-2,5-diméthanamine,
- N,N'-bis-(1,3,4-trihydroxybut-2-yl)-pyrazine-2,6-diméthanamine,
- N-méthyl-N-(1,3,4-trihydroxybut-2-yl)-N'-méthyl-N'-(1,3,4-trihydroxybut-2-yl)-pyrazine-2,5-diméthanamine,
- N-méthyl-N-(1,3,4-trihydroxybut-2-yl)-N'-méthyl-N'-(1,3,4-trihydroxybut-2-yl)-pyrazine-2,6-diméthanamine,
- N,N'-bis-[2-(hydroxyméthyl)-3-hydroxyprop-1-yl]-pyrazine-2,5-diméthanamine,
- N,N'-bis-[2-(hydroxyméthyl)-3-hydroxyprop-1-yl]-pyrazine-2,6-diméthanamine,
- N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,5-diméthanamine,
- N-méthyl-N-[2-(hydroxyméthyl)-3-hydroxyprop-1-yl]-N'-méthyl-N'-[2-(hydroxyméthyl)-3-hydroxyprop-1-yl]-pyrazine-2,6-diméthanamine,
- N,N,N',N'-tétrakis-[2-(hydroxyméthyl)-3-hydroxyprop-1-yl]-pyrazine-2,5-diméthanamine,
- N,N,N', N'-tétrakis-[2-(hydroxyméthyl)-3-hydroxyprop-1-yl]-pyrazine-2,6-diméthanamine,
- N,N'-bis-[1,1,1-tri-(hydroxyméthyl)-méthyl]-pyrazine-2,5-diméthanamine,
- N,N'-bis-[1,1,1-tri-(hydroxyméthyl)-méthyl]-pyrazine-2,6-diméthanamine,
- N-méthyl-N-[1,1,1-tri-(hydroxyméthyl)-méthyl]-N'-méthyl-N'-[1,1,1-tri-(hydroxyméthyl)-méthyl]-pyrazine-2,5-diméthanamine,
- N-méthyl-N-[1,1,1-tri-(hydroxyméthyl)-méthyl]-N'-méthyl-N'-[1,1,1-tri-(hydroxyméthyl)-méthyl]-pyrazine-2,6-diméthanamine,
- N,N'-bis-[1,1-di(hydroxyméthyl)-éth-1-yl]-pyrazine-2,5-diméthanamine,
- N,N'-bis-[1,1-di(hydroxyméthyl)-éth-1yl]-pyrazine-2,6-diméthanamine,
- N-méthyl-N-[1,1-di-(hydroxyméthyl)-éth-1-yl]-N'-méthyl-N'-[1,1-di-(hydroxyméthyl)-éth-1-yl]-pyrazine-2,5-diméthanamine,
- N-méthyl-N-[1,1-di-(hydroxyméthyl)-éth-1-yl]-N'-méthyl-N'-[1,1-di-(hydroxyméthyl)-éth-1-yl]-pyrazine-2,6-diméthanamine,
- 2,5-bis-(2-hydroxyéthyloxyméthyl)-pyrazine,
- 2,6-bis-(2-hydroxyéthyloxyméthyl)-pyrazine,
- 2.5-bis-[(2R)-2,3-dihydroxyprop-1-yloxyméthyl]-pyrazine,
- 2,5-bis-[(2S)-2,3-dihydroxyprop-1-yloxyméthyl]-pyrazine,
- 2,6-bis-[(2R)-2,3-dihydroxyprop-1-yloxyméthyl]-pyrazine,
- 2,6-bis-[(2S)-2,3-dihydroxyprop-1-yloxyméthyl]-pyrazine,
- 2,5-bis-(1,3-dihydroxyprop-2-yloxyméthyl)-pyrazine,
- 2,6-bis-(1,3-dihydroxyprop-2-yloxyméthyl]-pyrazine,
- 2,5-bis-(2,3,4-trihydroxybut-1-yloxyméthyl)-pyrazine,
- 2,6-bis-(2,3,4-trihydroxybut-1-yloxyméthyl)-pyrazine,
- 2,5-bis-(1,3,4-trihydroxybut-2-yloxyméthyl)-pyrazine,
- 2,6-bis-(1,3,4-trihydroxybut-2-yloxyméthyl)-pyrazine,
- 2,5-bis-[2-(hydroxyméthyl)-3-hydroxyprop-1-yloxyméthyl]-pyrazine,
- 2,6-bis-[2-(hydroxyméthyl)-3-hydroxyprop-1-yloxyméthyl]-pyrazine,
- 2,5-bis-[1,1,1-tri-(hydroxyméthyl)-méthyloxyméthyl]-pyrazine,
- 2,6-bis-[1,1,1-tri-(hydroxyméthyl)- méthyloxyméthyl]-pyrazine,
- 2,5-bis-[1,1-di(hydroxyméthyl)-éth-1-yloxyméthyl]-pyrazine,
- 2,6-bis-[1,1-di(hydroxyméthyl)-éth-1yloxyméthyl]-pyrazine,
- bis-N,N'-(2-hydroxyéthylamido)-2,5-pyrazine,
- bis-N,N'-(2-hydroxyéthylamido)-2,6-pyrazine,
- bis-[N-méthyl-(2-hydroxyéthylamido)]-2,5-pyrazine,
- bis-[N-méthyl-(2-hydroxyéthylamido)]-2,6-pyrazine,
- bis-(2,3-dihydroxyprop-1-ylamido)-2,5-pyrazine,
- bis-(2,3-dihydroxyprop-1-ylamido)-2,6-pyrazine
- bis-[N-méthyl-(2,3-dihydroxyprop-1-ylamido)]-2,5-pyrazine,
- bis-[N-méthyl-(2,3-dihydroxyprop-1-ylamido)]-2,6-pyrazine,
- bis-(N-méthyl-((1,3-dihydroxyprop-2-ylamido)-2,5-pyrazine,
- bis-(N-méthyl-((1,3-dihydroxyprop-2-ylamido)-2,6-pyrazine,
- bis-(2,3,4-trihydroxybut-1-ylamido)-2,5-pyrazine,
- bis-(2,3,4-trihydroxybut-1-ylamido)-2,6-pyrazine,
- bis-[N-méthyl-(2,3,4-trihydroxybut-1-yl amido)]-2,5-pyrazine,
- bis-[N-méthyl-(2,3,4-trihydroxybut-1-yl amido)]-2,6-pyrazine,
- bis-(1,3,4-trihydroxybut-2-ylamido)-2,5-pyrazine,
- bis-(1,3,4-trihydroxybut-2-ylamido)-2,6-pyrazine,
- bis-[N-méthyl-(1,3,4-trihydroxybut-2-yl amido)]-2,5-pyrazine,
- bis-[N-méthyl-(1,3,4-trihydroxybut-2-yl amido)]-2,6-pyrazine,
- bis-[2-(hydroxyméthyl)-3-hydroxyprop-1-ylamido]-2,5-pyrazine,
- bis-[2-(hydroxyméthyl)-3-hydroxyprop-1-ylamido]-2,6-pyrazine,
- bis-{[N-méthyl-[2-(hydroxyméthyl)-3-hydroxyprop-1-ylamido]}-2,5-pyrazine
- bis-{[N-méthyl-[2-(hydroxyméthyl)-3-hydroxyprop-1-ylamido]}-2,6-pyrazine
- bis-[1,1,1-tri-(hydroxyméthyl)-méthylamido]-2,5-pyrazine,
- bis-[1,1,1-tri-(hydroxyméthyl)-méthylamido]-2,6-pyrazine,
- bis-{[N-méthyl-[1,1,1-tri-(hydroxyméthyl)-méthylylamido]}-2,5-pyrazine
- bis-{[N-méthyl-[1,1,1-tri-(hydroxyméthyl)-méthylylamido]}-2,6-pyrazine
- bis-[1,1-di(hydroxyméthyl)-éth-1-ylamido]-2,5-pyrazine,
- bis-[1,1-di(hydroxyméthyl)-éth-1-ylamido]-2,6-pyrazine,
- bis-{[N-méthyl-[1,1-di-(hydroxyméthyl)-éth-1-ylamido]}-2,5-pyrazine
- bis-{[N-méthyl-[1,1-di-(hydroxyméthyl)-éth-1-ylamido]}-2,6-pyrazine
ou un stéréoisomère de ces composés ou un sel d'un tel composé avec un acide minéral ou organique pharmaceutiquement acceptable.

3. Médicaments contenant en tant que principe actif au moins un composé de formule (I) selon la revendication 1 dans laquelle
R₁ représente un radical -CO-NR₅R₆, -CO-N(CH₂-(CHOH)ₙ-CH₂OH)₂ dans lequel n est égal à 0, 1, 2, 3 ou 4, -CH₂-NR₅R₆, -CH₂-N(CH₂-(CHOH)_{y}-CH₂OH)₂ dans lequel y est égal à 0, 1, 2, 3 ou 4, et l'un des substituants R₃ ou R₄ représente un atome d'hydrogène et l'autre est identique à R₁, R₂ représente un atome d'hydrogène, R₅ représente un atome d'hydrogène ou un radical alkyle et R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃ ou,
alk représente un radical alkyle,
les radicaux alkyle contenant, sauf mention contraire, 1 à 6 atomes de carbone en chaîne droite ou ramifiée, leurs stéréoisomères et leurs sels avec un acide minéral ou organique.

4. Médicaments contenant en tant que principe actif un composé de formule (I) selon la revendication 1 choisi parmi les composés suivants :
- N,N'-bis-[(tris-(hydroxyméthyl)méthyl]-pyrazine-2,5-dicarboxamide
- N-méthyl-N-[(2S,3R,4R,5R)-2,3,4,5-tétrahydroxyhex-1-yl]-N'-méthyl-N'-[(2S,3R,4R,5R)-2,3,4,5-tetrahydroxyhex-1-yl]-pyrazine-2,5-dicarboxamide
- N,N'-bis-(1,3-dihydroxyprop-2-yl)-pyrazine-2,5-dicarboxamide
- N,N'-bis-(2,3-dihydroxyprop-1-yl)-pyrazine-2,5-dicarboxamide
- N,N,N',N'-tétrakis(2-hydroxyéthyl)-pyrazine-2,5-dicarboxamide
- bis-{N-[2(R,S)-3-dihydroxyprop-1-yl]-N-méthyl}-pyrazine-2,5-dicarboxamide
- N,N'-bis-(2-méthoxyéthyl)-pyrazine-2,5-dicarboxamide
- N,N'-bis-(2-hydroxyéthyloxyéthyl)-pyrazine-2,5-dicarboxamide
- N,N'-bis(1,3-dihydroxyprop-2-yl)-pyrazine-2,5-diméthanamine
- N,N,N',N'-tétrakis(2-hydroxyéthyl)-pyrazine-2,5-diméthanamine
- N,N'-bis-(1,3-dihydroxyprop-2-yl)-pyrazine-2,5-diméthanamine
- N-méthyl-N-[(2S,3R,4R,5R)-2,3,4,5-tétrahydoxyhex-1-yl]-N'-méthyl-N'[(2S,3R,4R,5R)-2,3,4,5-tétrahydroxyhex-1-yl]-pyrazine-2,5-diméthanamine
ou un sel d'un tel composé avec un acide minéral ou organique pharmaceutiquement acceptable.

5. Composés de formule : 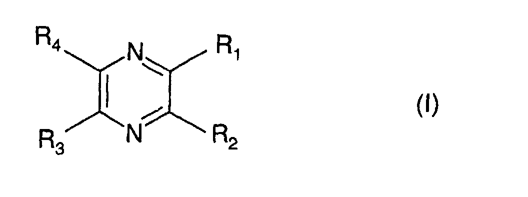 dans laquelle
R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ dans lequel n est égal à 0, 1, 2, 3 ou 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ dans lequel y est égal à 0, 1, 2, 3 ou 4, -CH₂-N(CH(CH₂OH)₂)₂,ou -CH₂-N(CH₂-CH(CH₂OH)₂)₂ et l'un des substituants R₃ ou R₄ représente un atome d'hydrogène et l'autre est identique à R₁ et R₂ représente un atome d'hydrogène,
R₅ représente un atome d'hydrogène ou un radical alkyle,
R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ ou -CH(CH₂OH)(CHOH)ₓ-CH₂OH dans lequel x est égal à 1, 2, 3 ou 4,
alk représente un radical alkyle.
les radicaux alkyle contenant, sauf mention contraire, 1 à 6 atomes de carbone en chaîne droite ou ramifiée, leurs stéréoisomères et leurs sels avec un acide minéral ou organique.

6. Composés de formule (I) selon la revendication 5 pour lesquels
R₁ représente un radical -CO-NR₅R₆, -CO-N(CH₂-(CHOH)ₙ-CH₂OH)₂ dans lequel n est égal à 0, 1, 2, 3 ou 4, -CH₂-NR₅R₆, -CH₂-N(CH₂-(CHOH)_{y}-CH₂OH)₂ dans lequel y est égal à 0, 1, 2, 3 ou 4, et l'un des substituants R₃ ou R₄ représente un atome d'hydrogène et l'autre est identique à R₁, R₂ représente un atome d'hydrogène, R₅ représente un atome d'hydrogène ou un radical alkyle et R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃,
alk représente un radical alkyle,
les radicaux alkyle contenant, sauf mention contraire, 1 à 6 atomes de carbone en chaîne droite ou ramifiée,
leurs stéréoisomères et leurs sels avec un acide minéral ou organique pharmaceutiquement acceptable.

7. Composés de formule (I) selon la revendication 5 choisis parmi les composés suivants :
- N,N'-bis-[(tris-(hydroxyméthyl)méthyl]-pyrazine-2,5-dicarboxamide
- N-méthyl-N-[(2S,3R,4R,5R)-2,3,4,5-tétrahydroxyhex-1-yl]-N'-méthyl-N'-[(2S,3R,4R,5R)-2,3,4,5-tetrahydroxyhex-1-yl]-pyrazine-2,5-dicarboxamide
- N,N'-bis-(1,3-dihydroxyprop-2-yl)-pyrazine-2,5-dicarboxamide
- N,N'-bis-(2,3-dihydroxyprop-1-yl)-pyrazine-2,5-dicarboxamide
- N,N,N',N'-tétrakis(2-hydroxyéthyl)-pyrazine-2,5-dicarboxamide
- bis-{N-[2(R,S)-3-dihydroxyprop-1-yl]-N-méthyl}-pyrazine-2,5-dicarboxamide
- N,N'-bis-(2-méthoxyéthyl)-pyrazine-2,5-dicarboxamide
- N,N'-bis-(2-hydroxyéthyloxyéthyl)-pyrazine-2,5-dicarboxamide
- N,N'-bis-(1,3-dihydroxyprop-2-yl)-pyridine-2,6-dicarboxamide
- N,N'-bis(1,3-dihydroxyprop-2-yl)-pyrazine-2,5-diméthanamine
- N,N,N',N'-tétrakis(2-hydroxyéthyl)-pyrazine-2,5-diméthanamine
- N,N'-bis-(1,3-dihydroxyprop-2-yl)-pyrazine-2,5-diméthanamine
- N-méthyl-N-[(2S,3R,4R,5R)-2,3,4,5-tétrahydoxyhex-1-yl]-N'-méthyl-N'[(2S,3R,4R,5R)-2,3,4,5-tétrahydroxyhex-1-yl]-pyrazine-2,5-diméthanamine et leurs sels avec un acide minéral ou organique.

8. Procédé de préparation des composé de formule (I) selon la revendication 5 pour lesquels R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, et l'un des substituants R₃ et R₄ représente un atome d'hydrogène et l'autre est identique à R₁ et R₂ représente un atome d'hydrogène **caractérisé en ce que** l'on fait réagir un dérivé de formule : 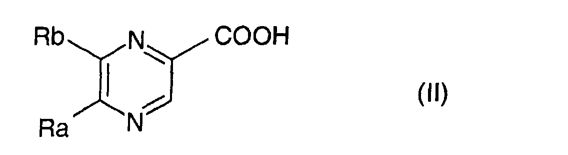 dans laquelle l'un des substituants Ra ou Rb représente un radical carboxy et l'autre un atome d'hydrogène, ou un dérivé réactif de ce diacide, sur une amine HNRcRd (III) dans laquelle soit Rc représente un atome d'hydrogène
ou un radical alkyle et Rd représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂ -CH₂-CH(CH₂OH)₂ ou -CH(CH₂OH)(CHOH)ₓ-CH₂OH dans lequel x est égal à 1, 2, 3 ou 4 et alk représente un radical alkyle, soit Rc et Rd sont identiques et représentent chacun un radical -CH₂-(CHOH)ₙ-CH₂OH dans lequel n est égal à 0, 1, 2, 3 ou 4, -CH(CH₂OH)₂, -CH₂OH ou -CH₂-CH(CH₂OH)₂, les radicaux alkyle contenant 1 à 6 atomes de carbone en chaîne droite ou ramifiée, isole le produit et le transforme éventuellement en sel avec un acide minéral ou organique.

9. Procédé de préparation des composés de formule (I) selon la revendication 5 pour lesquels R₁ représente un radical -CH₂-O-R₆ et l'un des substituants R₃ ou R₄ représente un atome d'hydrogène et l'autre est identique à R₁ et R₂ représente un atome d'hydrogène **caractérisé en ce que** l'on fait réagir un dérivé de formule : 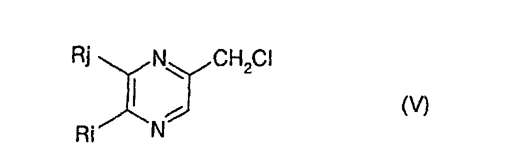 dans laquelle l'un des substituants Ri ou Rj représente un radical -CH₂Cl et l'autre un atome d'hydrogène sur un dérivé R₆OH (VI) pour lequel R₆ a les mêmes significations que dans la revendication 1, isole le produit et le transforme éventuellement en sel avec un acide minéral ou organique.

10. Procédé de préparation des composés de formule (I) selon la revendication 5 pour lesquels R₁ représente un radical -CH₂-NR₅R₆, -CH₂-N(CH₂OH)₂, -CH₂-N(CH₂-(CHOH)_{y}-CH₂OH)₂, -CH₂-N(CH(CH₂OH)₂)₂ ou -CH₂-N(CH₂-CH(CH₂OH)₂)₂ et l'un des substituants R₃ ou R₄ représente un atome d'hydrogène et l'autre est identique à R₁ et R₂ représente un atome d'hydrogène **caractérisé en ce que** l'on fait réagir un dérivé de formule : 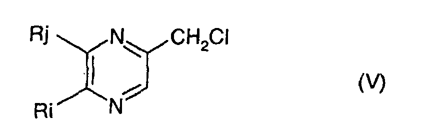 dans laquelle l'un des substituants Ri et Rj représente un radical -CH₂Cl et l'autre un atome d'hydrogène sur une amine HNRcRd (III) dans laquelle soit Rc représente un atome d'hydrogène ou un radical alkyle et Rd représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ ou -CH(CH₂OH)(CHOH)ₓ-CH₂OH dans lequel x est égal à 1, 2, 3 ou 4 soit Rc et Rd sont identiques et représentent un radical -CH₂OH, -CH₂-(CHOH)_{y}-CH₂OH dans lequel y est égal à 0, 1, 2, 3 ou 4 ou -CH₂-CH(CH₂OH)₂, isole le produit et le transforme éventuellement en sel avec un acide minéral ou organique.

11. Procédé de préparation des composés de formule (I) selon la revendication 5 pour lesquels R₁ représente un radical -NR₅-CO-R₆ et l'un des substituants R₃ ou R₄ représente un atome d'hydrogène et l'autre est identique à R₁ et R₂ représente un atome d'hydrogène **caractérisé en ce que** l'on fait réagir un dérivé de formule : 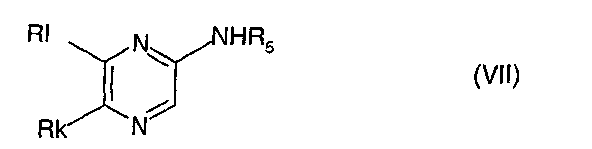 dans laquelle R₅ a les mêmes significations que dans la revendication 1 et l'un des substituants Rk ou RI représente un atome d'hydrogène et l'autre un radical NHR₅ dans lequel R₅ a les mêmes significations que dans la revendication 1, sur un dérivé HOOC-R₆ (VIII) pour lequel R₆ a les mêmes significations que dans la revendication 1 ou un dérivé réactif de cet acide, isole le produit et le transforme éventuellement en sel avec un acide minéral ou organique.

12. Procédé de préparation des composés de formule (I) selon la revendication 5 pour lesquels R₁ représente un radical -CH₂-O-R₆ dans lequel R₆ représente un radical -alk-O-alk et l'un des substituants R₃ ou R₄ représente un atome d'hydrogène et l'autre est identique à R₁ et R₂ représente un atome d'hydrogène **caractérisé en ce que** l'on fait réagir un dérivé de formule : 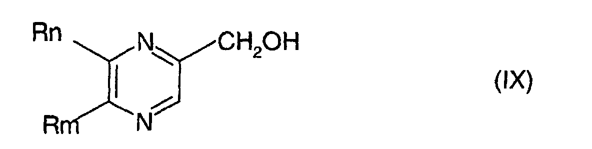 dans laquelle l'un des substituants Rm ou Rn représente un radical -CH₂OH et l'autre un atome d'hydrogène sur un dérivé Hal-alk-O-alk (X) pour lequel alk représente un radical alkyle et Hal représente un atome d'halogène et, de préférence, un atome de chlore ou de brome, isole le produit et le transforme éventuellement en sel avec un acide minéral ou organique.

13. Utilisation d'un composé de formule 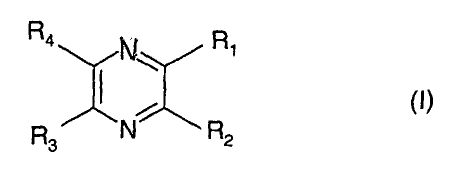 dans laquelle
R₁ représente un radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ dans lequel n est égal à 0, 1, 2, 3 ou 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ dans lequel y est égal à 0, 1, 2, 3 ou 4, -CH₂-N(CH(CH₂OH)₂)₂ ou -CH₂-N(CH₂-CH(CH₂OH)₂)₂ et l'un des substituants R₃ ou R₄ représente un atome d'hydrogène et l'autre est identique à R₁ et R₂ représente un atome d'hydrogène,
R₅ représente un atome d'hydrogène ou un radical alkyle,
R₆ représente un radical -CH₂-(CHOH)m-CH₂OH dans lequel m est égal à 0, 1, 2, 3 ou 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ ou -CH(CH₂OH)-(CHOH)ₓ-CH₂OH dans lequel x est égal à 1, 2, 3 ou 4,
alk représente un radical alkyle,
les radicaux alkyle contenant, sauf mention contraire, 1 à 6 atomes de carbone en chaîne droite ou ramifiée, leurs stéréoisomères et leurs sels avec un acide minéral ou organique, pour la préparation d'un médicament utile pour le traitement ou la prévention du diabète et des complications du diabète.

## Claims

1. Medicaments containing, as active principle, at least one compound of formula: 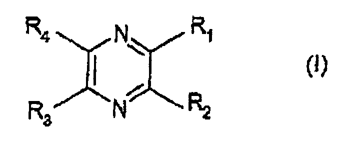 in which
R₁ represents a radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ in which n is equal to 0, 1, 2, 3, or 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ in which y is equal to 0, 1, 2, 3 or 4, -CH₂-N(CH(CH₂OH)₂)₂ or -CH₂-N(CH₂-CH(CH₂OH)₂)₂ and one of the substituents R₃ or R₄ represents a hydrogen atom and the other is identical to R₁ and R₂ represents a hydrogen atom,
R₅ represents a hydrogen atom or an alkyl radical,
R₆ represents a radical -CH₂-(CHOH)m-CH₂OH in which m is equal to 0, 1, 2, 3 or 4, -alk-O-alk-CH₂OH, -alk-O-alk, -CH(CH₂OH)₂, -C(CH₂OH)₃, -C(CH₃) (CH₂OH)₂, -CH₂-CH(CH₂OH)₂ or -CH(CH₂OH)-(CHOH)ₓ-CH₂OH in which x is equal to 1, 2, 3 or 4,
alk represents an alkyl radical,
the alkyl radicals containing, unless otherwise indicated, 1 to 6 carbon atoms in a straight or branched chain, their stereoisomers and their salts with an inorganic or organic acid.

2. Medicaments containing a compound of formula (I) according to Claim 1, chosen from the following compounds:
-N,N'-bis(2-hydroxyethyl)pyrazine-2,5-dicarboxamide,
-N,N'-bis(2-hydroxyethyl)pyrazine-2,6-dicarboxamide,
-N-methyl-N-(2-hydroxyethyl-N'-methyl-N'(2-hydroxy-ethyl)pyrazine-2,5-dicarboxamide,
-N-methyl-N-(2-hydroxyethyl)-N'-methyl-N'(2-hydroxy-ethyl)pyrazine-2,6-dicarboxamide,
-N,N,N',N'-tetrakis(2-hydroxyethyl)pyrazine-2,5-dicarboxamide,
-N,N,N',N'-tetrakis(2-hydroxyethyl)pyrazine-2,6-dicarboxamide,
-N,N'-bis(2,3-dihydroxy-1-propyl)pyrazine-2,5-dicarboxamide,
-N,N'-bis(2,3-dihydroxy-1-propyl)pyrazine-2,6-dicarboxamide,
-N-methyl-N-(2,3-dihydroxy-1-propyl)-N'-methyl-N'-(2,3-dihydroxy-1-propyl)pyrazine-2,5-dicarboxamide,
-N-methyl-N-(2,3-dihydroxy-1-propyl)-N'-methyl-N'-(2,3-dihydroxy-1-propyl)pyrazine-2,6-dicarboxamide,
-N,N,N',N'-tetrakis(2,3-dihydroxy-1-propyl)pyrazine-2,5-dicarboxamide,
-N',N,N',N'-tetrakis(2,3-dihydroxy-1-propyl)pyrazine-2,6-dicarboxamide,
-N,N'-bis(1,3-dihydroxy-2-propyl)pyrazine-2,5-dicarboxamide,
-N,N'-bis(1,3-dihydroxy-2-propyl)pyrazine-2,6-dicarboxamide,
-N-methyl-N-(1,3-dihydroxy-2-propyl)-N'-methyl-N'-(1,3-dihydroxy-2-propyl)pyrazine-2,5-dicarboxamide,
-N-methyl-N-(1,3-dihydroxy-2-propyl)-N'-methyl-N'-(1,3-dihydroxy-2-propyl)pyrazine-2,6-dicarboxamide,
-N,N,N',N'-tetrakis(1,3-dihydroxy-2-propyl)pyrazine-2,5-dicarboxamide,
-N,N,N',N'-tetrakis(1,3-dihydroxy-2-propyl)pyrazine-2,6-dicarboxamide,
-N,N'-bis(2,3,4-trihydroxy-1-butyl)pyrazine-2,5-dicarboxamide,
-N,N'-bis(2,3,4-trihydroxy-1-butyl)pyrazine-2,6-dicarboxamide,
-N-methyl-N-(2,3,4-trihydroxy-1-butyl)-N'-methyl-N'-(2,3,4-trihydroxy-1-butyl)pyrazine-2,5-dicarboxamide,
-N-methyl-N-(2,3,4-trihydroxy-1-butyl)-N'-methyl-N'-(2,3,4-trihydroxy-1-butyl)pyrazine-2,6-dicarboxamide,
-N,N'-bis(1,3,4-trihydroxy-2-butyl)pyrazine-2,5-dicarboxamide,
-N,N'-bis(1,3,4-trihydroxy-2-butyl)pyrazine-2,6-dicarboxamide,
-N-methyl-N-(1,3,4-trihydroxy-2-butyl)-N'-methyl-N'-(1,3,4-trihydroxy-2-butyl)pyrazine-2,5-dicarboxamide,
-N-methyl-N-(1,3,4-trihydroxy-2-butyl)-N'-methyl-N'-(1,3,4-trihydroxy-2-butyl)pyrazine-2,6-dicarboxamide,
-N,N'-bis[2-(hydroxymethyl)-3-hydroxy-1-propyl]-pyrazine-2,5-dicarboxamide,
-N,N'-bis[2-(hydroxymethyl) -3-hydroxy-1-propyl]- : pyrazine-2,6-dicarboxamide,
-N-methyl-N-[2-(hydroxymethyl)-3-hydroxy-1-propyl]-N'-methyl-N'-[2-(hydroxymethyl)-3-hydroxy-1-propyl]-pyrazine-2,5-dicarboxamide,
-N-methyl-N-[2-(hydroxymethyl)-3-hydroxy-1-propyl]-N'-methyl-N'-[2-(hydroxymethyl)-3-hydroxy-1-propyl]-pyrazine-2,6-dicarboxamide,
-N,N,N',N'-tetrakis[2-(hydroxymethyl)-3-hydroxy-1-propyl]pyrazine-2,5-dicarboxamide,
-N,N,N',N'-tetrakis[2-(hydroxymethyl)-3-hydroxy-1-propyl]pyrazine-2,6-dicarboxamide,
-N,N'-bis[1,1,1-tri(hydroxymethyl)methyl]pyrazine-2,5-dicarboxamide,
-N,N'-bis[1,1,1-tri(hydroxymethyl)methyl]pyrazine-2,6-dicarboxamide,
-N-methyl-N-[1,1,1-tri(hydroxymethyl)methyl]-N'-methyl-N'-[1,1,1-tri(hydroxymethyl)methyl]pyrazine-2,5-dicarboxamide,
-N-methyl-N-[1,1,1-tri(hydroxymethyl)methyl]-N'-methyl-N'-[1,1,1-tri(hydroxymethyl)methyl]pyrazine-2,6-dicarboxamide,
-N,N'-bis[1,1-di(hydroxymethyl)-1-ethyl]pyrazine-2,5-dicarboxamide,
-N,N'-bis[1,1-di(hydroxymethyl)-1-ethyl]pyrazine-2,6-dicarboxamide,
-N-methyl-N-[1,1-di(hydroxymethyl)-1-ethyl]-N'-methyl-N'-[1,1-di(hydroxymethyl)-1-ethyl]pyrazine-2,5-dicarboxamide,
-N-methyl-N-[1,1-di(hydroxymethyl)-1-ethyl]-N'-methyl-N'-[1,1-di(hydroxymethyl)-1-ethyl]pyrazine-2,6-dicarboxamide,
-N,N,N',N'-tetrakis(1,3-dihydroxy-2-propyl)pyrazine-2,5-dicarboxamide,
-N,N,N',N'-tetrakis(1,3-dihydroxy-2-propyl)pyrazine-2,6-dicarboxamide,
-N,N'-bis(2-hydroxyethyl)pyrazine-2, 5-dimethanamine,
-N,N'-bis(2-hydroxyethyl)pyrazine-2, 6-dimethanamine,
-N-methyl-N-(2-hydroxyethyl)-N'-methyl-N'-(2-hydroxy-ethyl)pyrazine-2,5-dimethanamine,
-N-methyl-N-(2-hydroxyethyl)-N'-methyl-N'-(2-hydroxy-ethyl)pyrazine-2,6-dimethanamine,
-N,N,N',N'-tetrakis(2-hydroxyethyl)pyrazine-2,5-dimethanamine,
-N,N,N',N'-tetrakis(2-hydroxyethyl)pyrazine-2,6-dimethanamine,
-N,N'-bis(2,3-dihydroxy-1-propyl)pyrazine-2,5-dimethanamine,
-N,N'-bis(2,3-dihydroxy-1-propyl)pyrazine-2,6-dimethanamine,
-N-methyl-N-(2,3-dihydroxy-1-propyl)-N'-methyl-N'-(2,3-dihydroxy-1-propyl)pyrazine-2,5-dimethanamine,
-N-methyl-N-(2,3-dihydroxy-1-propyl)-N'-methyl-N'-(2,3-dihydroxy-1-propyl)pyrazine-2,6-dimethanamine,
-N,N,N',N'-tetrakis(2,3-dihydroxy-1-propyl)pyrazine-2,5-dimethanamine,
-N,N,N',N'-tetrakis(2,3-dihydroxy-1-propyl)pyrazine-2,6-dimethanamine,
-N,N'-bis(1,3-dihydroxy-2-propyl)pyrazine-2,5-dimethanamine,
-N,N'-bis(1,3-dihydroxy-2-propyl)pyrazine-2,6-dimethanamine,
-N-methyl-N-(1,3-dihydroxy-2-propyl)-N'-methyl-N'-(1,3-dihydroxy-2-propyl)pyrazine-2,5-dimethanamine,
-N-methyl-N-(1,3-dihydroxy-2-propyl)-N'-methyl-N'-(1,3-dihydroxy-2-propyl)pyrazine-2,6-dimethanamine,
-N,N,N',N'-tetrakis(1,3-dihydroxy-2-propyl)pyrazine-2,5-dimethanamine,
-N,N,N',N'-tetrakis(1,3-dihydroxy-2-propyl)pyrazine-2,6-dimethanamine,
-N,N'-bis(2,3,4-trihydroxy-1-butyl)pyrazine-2,5-dimethanamine,
-N,N'-bis(2,3,4-trihydroxy-1-butyl)pyrazine-2,6-dimethanamine,
-N-methyl-N-(2,3,4-trihydroxy-1-butyl)-N'-methyl-N'-(2,3,4-trihydroxy-1-butyl)pyrazine-2,5-dimethanamine,
-N-methyl-N-(2,3,4-trihydroxy-1-butyl)-N'-methyl-N'-(2,3,4-trihydroxy-1-butyl)pyrazine-2,6-dimethanamine,
-N,N'-bis(1,3,4-trihydroxy-2-butyl)pyrazine-2,5-dimethanamine,
-N,N'-bis(1,3,4-trihydroxy-2-butyl)pyrazine-2,6-dimethanamine,
-N-methyl-N-(1,3,4-trihydroxy-2-butyl)-N'-methyl-N'-(1,3,4-trihydroxy-2-butyl)pyrazine-2,5-dimethanamine,
-N-methyl-N-(1,3,4-trihydroxy-2-butyl)-N'-methyl-N'-(1,3,4-trihydroxy-2-butyl)pyrazine-2,6-dimethanamine,
-N,N'-bis[2-(hydroxymethyl)-3-hydroxy-1-propyl]-pyrazine-2,5-dimethanamine,
-N,N'-bis[2-(hydroxymethyl)-3-hydroxy-1-propyl]-pyrazine-2,6-dimethanamine,
-N-methyl-N-[2-(hydroxymethyl)-3-hydroxy-1-propyl]-N'-methyl-N' [2-(hydroxymethyl)-3-hydroxy-1-propyl]-pyrazine-2,5-dimethanamine,
-N-methyl-N-[2-(hydroxymethyl)-3-hydroxy-1-propyl]-N'-methyl-N'-[2-(hydroxymethyl)-3-hydroxy-1-propyl]-pyrazine-2,6-dimethanamine,
-N,N,N',N'-tetrakis[2-(hydroxymethyl)-3-hydroxy-1-propyl]pyrazine-2,5-dimethanamine,
-N,N,N',N'-tetrakis[2-(hydroxymethyl)-3-hydroxy-1-propyl]pyrazine-2,6-dimethanamine,
-N,N'-bis[1,1,1-tri(hydroxymethyl)methyl]pyrazine-2,5-dimethanamine,
-N,N'-bis[1,1,1-tri(hydroxymethyl)methyl]pyrazine-2,6-dimethanamine,
-N-methyl-N-[1,1,1-tri(hydroxymethyl)methyl]-N'-methyl-N'-[1,1,1-tri(hydroxymethyl)methyl]pyrazine-2,5-dimethanamine,
-N-methyl-N-[1,1,1-tri(hydroxymethyl)methyl]-N'-methyl-N'-[1,1,1,tri(hydroxymethyl)methyl]pyrazine-2,6-dimethanamine,
-N,N'-bis[1,1-di(hydroxymethyl)-1-ethyl]pyrazine-2,5-dimethanamine,
-N,N'-bis[1,1-di(hydroxymethyl)-1-ethyl]pyrazine-2,6-dimethanamine,
-N-methyl-N-[1,1-di(hydroxymethyl)-1-ethyl]-N'-methyl-N'-[1,1-di(hydroxymethyl)-1-ethyl]pyrazine-2,5-dimethanamine,
-N-methyl-N-[1,1-di(hydroxymethyl)-1-ethyl]-N'-methyl-N'-[1,1-di(hydroxymethyl)-1-ethyl]pyrazine-2,6-dimethanamine,
-2,5-bis(2-hydroxyethyloxymethyl)pyrazine,
-2,6-bis(2-hydroxyethyloxymethyl)pyrazine,
-2,5-bis[(2R)-2,3-dihydroxy-1-propyloxymethyl]pyrazine,
-2,5-bis[(2S)-2,3-dihydroxy-1-propyloxymethyl]pyrazine,
-2,6-bis[(2R)-2,3-dihydroxy-1-propyloxymethyl]pyrazine,
-2,6-bis[(2S)-2,3-dihydroxy-1-propyloxymethyl]pyrazine,
-2,5-bis(1,3-dihydroxy-2-propyloxymethyl)pyrazine,
-2,6-bis(1,3-dihydroxy-2-propyloxymethyl]pyrazine,
-2,5-bis(2,3,4-trihydroxy-1-butyloxymethyl)pyrazine,
-2,6-bis(2,3,4-trihydroxy-1-butyloxymethyl)pyrazine,
-2,5-bis(1,3,4-trihydroxy-2-butyloxymethyl)pyrazine,
-2,6-bis(1,3,4-trihydroxy-2-butyloxymethyl)pyrazine,
-2,5-bis[2-(hydroxyethyl)-3-hydroxy-1-propyloxy-methyl]pyrazine,
-2,6-bis[2-(hydroxymethyl)-3-hydroxy-1-propyloxy-methyl]pyrazine,
-2,5-bis[1,1,1-tri(hydroxymethyl)methyloxymethyl]-pyrazine,
-2,6-bis[1,1,1-tri(hydroxymethyl)methyloxymethyl]-pyrazine,
-2,5-bis[1,1-di(hydroxymethyl)-1-ethyloxymethyl]-pyrazine,
-2,6-bis[1,1-di(hydroxymethyl)-1-ethyloxymethyl]-pyrazine,
-bis-N,N'-(2-hydroxyethylamido)-2,5-pyrazine,
-bis-N,N'-(2-hydroxyethylamido)-2,6-pyrazine,
-bis[N-methyl(2-hydroxyethylamido)]-2,5-pyrazine,
-bis[N-methyl(2-hydroxyethylamido)]-2,6-pyrazine,
-bis(2,3-dihydroxy-1-propylamido)-2,5-pyrazine,
-bis(2,3-dihydroxy-1-propylamido)-2,6-pyrazine,
-bis[N-methyl(2,3-dihydroxy-1-propylamido)]-2,5-pyrazine,
-bis[N-methyl(2,3-dihydroxy-1-propylamido)] -2,6-pyrazine,
-bis[N-methyl-(1,3-dihydroxy-2-propylamido)]-2,5-pyrazine,
-bis[N-methyl(1,3-dihydroxy-2-propylamido)]-2,6-pyrazine,
-bis(2,3,4-trihydroxy-1-butylamido)-2,5-pyrazine,
-bis(2,3,4-trihydroxy-1-butylamido)-2,6-pyrazine,
-bis[N-methyl(2,3,4-trihydroxy-1-butylamido)]-2,5-pyrazine,
-bis[N-methyl(2,3,4-trihydroxy-1-butylamido)]-2,6-pyrazine,
-bis(1,3,4-trihydroxy-2-butylamido)-2,5-pyrazine,
-bis(1,3,4-trihydroxy-2-butylamido)-2,6-pyrazine,
-bis[N-methyl(1,3,4-trihydroxy-2-butylamido)]-2,5-pyrazine.
-bis[N-methyl(1,3,4-trihydroxy-2-butylamido)]-2,6-pyrazine,
-bis[2-(hydroxymethyl)-3-hydroxy-1-propylamido]-2,5-pyrazine,
-bis[2-(hydroxymethyl)-3-hydroxy-1-propylamido]-2,6-pyrazine,
-bis{[N-methyl[2-(hydroxymethyl)-3-hydroxy-1-propyl-amido]}-2,5-pyrazine,
-bis{[N-methyl[2-(hydroxymethyl)-3-hydroxy-1-propyl-amido]}-2,6-pyrazine,
-bis[1,1,1-tri(hydroxymethyl)methylamido]-2,5-pyrazine,
-bis[1,1,1-tri(hydroxymethyl)methylamido]-2,6-pyrazine,
-bis{[N-methyl[1,1,1-tri(hydroxymethyl)methylamido]}-2,5-pyrazine,
-bis{[N-methyl[1,1,1-tri(hydroxymethyl)methylamido]}-2,6-pyrazine,
-bis[1,1-di(hydroxymethyl)-1-ethylamido]-2,5-pyrazine,
-bis[1,1-di(hydroxymethyl)-1-ethylamido]-2,6-pyrazine,
-bis{[N-methyl[1,1-di(hydroxymethyl)-1-ethylamido]}-2,5-pyrazine,
-bis{[N-methyl[1,1-di(hydroxymethyl)-1-ethylamido]}-2,6-pyrazine,
or a stereoisomer of these compounds or a salt of such a compound with a pharmaceutically acceptable inorganic or organic acid.

3. Medicaments containing, as active principle, at least one compound of formula (I) according to Claim 1, in which
R₁ represents a radical -CO-NR₅R₆, -CO-N(CH₂-(CHOH)ₙ-CH₂OH)₂ in which n is equal to 0, 1, 2, 3 or 4, -CH₂-NR₅R₆, -CH₂-N(CH₂-(CHOH)_{y}-CH₂OH)₂ in which y is equal to 0, 1, 2, 3 or 4, and one of the substibuents R₃ or R₄ represents a hydrogen atom and the other is identical to R₁, R₂ represents a hydrogen atom, R₅ represents a hydrogen atom or an alkyl radical and R₆ represents a radical -CH₂-(CHOH)m-CH₂OH in which m is equal to 0, 1, 2, 3 or 4, -alk-O-alk-CH₂OH, -alk-O-alk, -CH(CH₂OH)₂ or -C(CH₂OH)₃,
alk represents an alkyl radical,
the alkyl radicals containing, unless otherwise indicated, 1 to 6 carbon atoms in a straight or branched chain, their stereoisomers and their salts with a pharmaceutically acceptable inorganic or organic acid.

4. Medicaments containing, as active principle, a compound of formula (I) according to Claim 1, chosen from the following compounds:
-N,N'-bis[(tris(hydroxymethyl)methyl]pyrazine-2,5-dicarboxamide,
-N-methyl-N-[(2S,3R,4R,5R)-2,3,4,5-tetrahydroxy-1-hexyl]-N'-methyl-N'-[(2S,3R,4R,5R)-2,3,4,5-tetra-hydroxy-1-hexyl]pyrazine-2,5-dicarboxamide,
-N,N'-bis(1,3-dihydroxy-2-propyl)pyrazine-2,5-dicarboxamide,
-N,N'-bis(2,3-dihydroxy-1-propyl)pyrazine-2,5-dicarboxamide,
-N,N,N',N'-tetrakis(2-hydroxyethyl)pyrazine-2,5-dicarboxamide,
-bis{N-[2(R,S)-3-dihydroxy-1-propyl]-N-methyl}pyrazine-2,5-dicarboxamide,
-N,N'-bis(2-methoxyethyl)pyrazine-2,5-dicarboxamide,
-N,N'-bis(2-hydroxyethyloxyethyl)pyrazine-2,5-dicarboxamide,
-N,N'-bis(1,3-dihydroxy-2-propyl)pyrazine-2,5-dimethanamine,
-N,N,N',N'-tetrakis(2-hydroxyethyl)pyrazine-2,5-dimethanamine,
-N,N'-bis(1,3-dihydroxy-2-propyl)pyrazine-2,5-dimethanamine,
-N-methyl-N-[(2S,3R,4R,5R)-2,3,4,5-tetrahydroxy-1-hexyl]-N'-methyl-N'-[(2S,3R,4R,5R)-2,3,4,5-tetra-hydroxy-1-hexyl]pyrazine-2,5-dimethanamine,
or a salt of such a compound with a pharmaceutically acceptable inorganic or organic acid.

5. Compounds of formula: 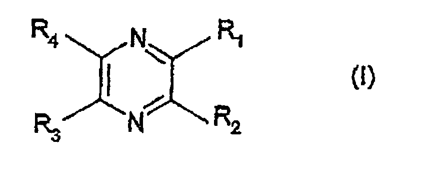 in which
R₁ represents a radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ in which n is equal to 0, 1, 2, 3 or 4, -CH₂-O-R₅, -NR₅-CO-R₆, -CH₂-NR₅R₆, -CH₂-N(CH₂OH)₂, -CH₂-N(CH₂-(CHOH)_{y}-CH₂OH)₂ in which y is equal to 0, 1, 2, 3 or 4, -CH₂-N(CH(CH₂OH)₂)₂ or -CH₂-N(CH₂-CH(CH₂OH)₂)₂ and one of the substituents R₃ or R₄ represents a hydrogen atom and the other is identical to R₁ and R₂ represents a hydrogen atom,
R₅ represents a hydrogen atom or an alkyl radical,
R₆ represents a radical -CH₂-(CHOH)m-CH₂OH in which m is equal to 0, 1, 2, 3 or 4, -alk-O-alk-CH₂OH, -alk-O-alk, -CH(CH₂OH)₂, -C(CH₂OH)₃, -C(CH₃) (CH₂OH)₂, -CH₂-CH(CH₂OH)₂, or -CH(CH₂OH) (CHOH)ₓ-CH₂OH in which x is equal to 1, 2, 3 or 4,
alk represents an alkyl radical,
the alkyl radicals containing, unless otherwise indicated, 1 to 6 carbon atoms in a straight or branched chain, their stereoisomers and their salts with an inorganic or organic acid.

6. Compounds of formula (I) according to Claim 5, for which
R₁ represents a radical -CO-NR₅R₆, -CO-N(CH₂-(CHOH)ₙ-CH₂OH)₂ in which n is equal to 0, 1, 2, 3 or 4, -CH₂-NR₅R₆, -CH₂-N(CH₂-(CHOH)_{y}-CH₂OH)₂ in which y is equal to 0, 1, 2, 3 or 4, and one of the substituents R₃ or R₄ represents a hydrogen atom and the other is identical to R₁, R₂ represents a hydrogen atom, R₅ represents a hydrogen atom or an alkyl radical and R₆ represents a radical -CH₂-(CHOH)m-CH₂OH in which m is equal to 0, 1, 2, 3 or 4, -alk-O-alk-CH₂OH, -alk-O-alk, -CH(CH₂OH)₂ or -C(CH₂OH)₃,
alk represents an alkyl radical,
the alkyl radicals containing, unless otherwise indicated, 1 to 6 carbon atoms in a straight or branched chain,
their stereoisomers and their salts with a pharmaceutically acceptable inorganic or organic acid.

7. Compounds of formula (I) according to Claim 5, chosen from the following compounds:
-N,N'-bis[(tris(hydroxymethyl)methyl]pyrazine-2,5-dicarboxamide,
-N-methyl-N-[(2S,3R,4R,5R)-2,3,4,5-tetrahydroxy-1-hexyl]-N'-methyl-N'-[(2S,3R,4R,5R)-2,3,4,5-tetrahydroxy-1-hexyl]pyrazine-2,5-dicarboxamide,
-N,N'-bis(1,3-dihydroxy-2-propyl)pyrazine-2,5-dicarboxamide,
-N,N'-bis(2,3-dihydroxy-1-propyl)pyrazine-2,5-dicarboxamide,
-N,N,N',N'-tetrakis(2-hydroxyethyl)pyrazine-2,5-dicarboxamide,
-bis{N-[2(R,S)-3-dihydroxy-1-propyl]-N-methyl}pyrazine-2,5-dicarboxamide,
-N,N'-bis(2-methoxyethyl)pyrazine-2,5-dicarboxamide,
-N,N'-bis(2-hydroxyethyloxyethyl)pyrazine-2,5-dicarboxamide,
-N,N'-bis(1,3-dihydroxy-2-propyl)pyridine-2,6-dicarboxamide,
-N,N'-bis(1,3-dihydroxy-2-propyl)pyrazine-2,5-dimethanamine,
-N,N,N',N'-tetrakis(2-hydroxyethyl)pyrazine-2,5-dimethanamine,
-N,N'-bis(1,3-dihydroxy-2-propyl)pyrazine-2,5-dimethanamine,
-N-methyl-N-[(2S,3R,4R,5R)-2,3,4,5-tetrahydroxy-1-hexyl]-N'-methyl-N'-[(2S,3R,4R,5R)-2,3,4,5-tetrahydroxy-1-hexyl]pyrazine-2,5-dimethanamine,
and their salts with an inorganic or organic acid.

8. Process for the preparation of the compounds of formula (I) according to Claim 5, for which R₁ represents a radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, and one of the substituents R₃ and R₄ represents a hydrogen atom and the other is identical to R₁ and R₂ represents a hydrogen atom, **characterized in that** a derivative of formula: 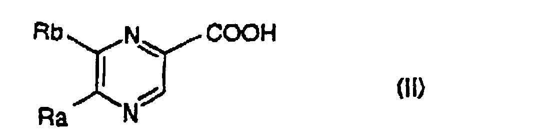 in which one of the substituents Ra or Rb represents a carboxyl radical and the other represents a hydrogen atom, or a reactive derivative of this diacid, is reacted with an amine HNRcRd (III) in which either Rc represents a hydrogen atom or an alkyl radical and Rd represents a radical -CH₂-(CHOH)m-CH₂OH in which m is equal to 0, 1, 2, 3 or 4, -alk-O-alk-CH₂OH, -alk-O-alk, -CH(CH₂OH)₂, -C(CH₂OH)₃, -C(CH₃) (CH₂OH)₂, -CH₂-CH(CH₂OH)₂ or -CH(CH₂OH) (CHOH)ₓ-CH₂OH in which x is equal to 1, 2, 3 or 4 and alk represents an alkyl radical, or Rc and Rd are identical and each represent a radical -CH₂-(CHOH)ₙ-CH₂OH in which n is equal to 0, 1, 2, 3 or 4, -CH(CH₂OH)₂, -CH₂OH or -CH₂-CH(CH₂OH)₂, the alkyl radicals containing 1 to 6 carbon atoms in a straight or branched chain, and the product is isolated and optionally converted into a salt with an inorganic or organic acid.

9. Process for the preparation of the compounds of formula (I) according to Claim 5, for which R₁ represents a radical -CH₂-O-R₆ and one of the substituents R₃ or R₄ represents a hydrogen atom and the other is identical to R₁ and R₂ represents a hydrogen atom, **characterized in that** a derivative of formula: 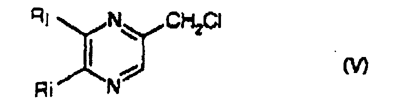 in which one of the substituents Ri or Rj represents a radical -CH₂Cl and the other represents a hydrogen atom, is reacted with a derivative R₆OH (VI) for which R₆ has the same meanings as in Claim 1, and the product is isolated and optionally converted into a salt with an inorganic or organic acid.

10. Process for the preparation of the compounds of formula (I) according to Claim 5, for which Rᵢ represents a radical -CH₂-NR₅R₆, -CH₂-N(CH₂OH)₂, -CH₂-N(CH₂-(CHOH)_{y}-CH₂OH)₂, -CH₂-N(CH(CH₂OH)₂)₂ or -CH₂-N(CH₂-CH(CH₂OH)₂)₂ and one of the substituents R₃ or R₄ represents a hydrogen atom and the other is identical to R₁, and R₂ represents a hydrogen atom, **characterized in that** a derivative of formula: 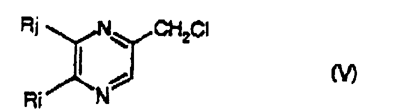 in which one of the substituents Ri and Rj represents a -CH₂Cl radical and the other represents a hydrogen atom is reacted with an amine HNRcRd (III) in which either Rc represents a hydrogen atom or an alkyl radical and Rd represents a radical -CH₂-(CHOH)m-CH₂OH in which m is equal to 0, 1, 2, 3 or 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ or -CH(CH₂OH) (CHOH)ₓ-CH₂OH in which x is equal to 1, 2, 3 or 4, or Rc and Rd are identical and represent a radical -CH₂OH, -CH₂-(CHOH)_{y}-CH₂OH in which y is equal to 0, 1, 2, 3 or 4, or -CH₂-CH(CH₂OH)₂, and the product is isolated and optionally converted into a salt with an inorganic or organic acid.

11. Process for the preparation of the compounds of formula (I) according to Claim 5, for which R₁ represents a radical -NR₅-CO-R₆ and one of the substituents R₃ or R₄ represents a hydrogen atom and the other is identical to R₁, and R₂ represents a hydrogen atom, **characterized in that** a derivative of formula: 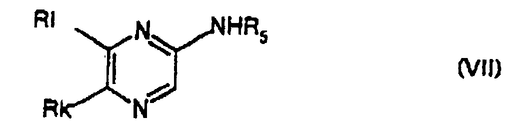 in which R₅ has the same meanings as in Claim 1 and one of the substituents Rk or Rl represents a hydrogen atom and the other represents a radical NHR₅ in which R₅ has the same meanings as in Claim 1, is reacted with a derivative HOOC-R₆ (VIII) in which R₆ has the same meanings as in Claim 1 or a reactive derivative of this acid, and the product is isolated and optionally converted into a salt with an inorganic or organic acid.

12. Process for the preparation of the compounds of formula (I) according to Claim 5, for which R₁ represents a radical -CH₂-O-R₆ in which R₆ represents a radical -alk-O-alk and one of the substituents R₃ or R₄ represents a hydrogen atom and the other is identical to R₁, and R₂ represents a hydrogen atom, **characterized in that** a derivative of formula: 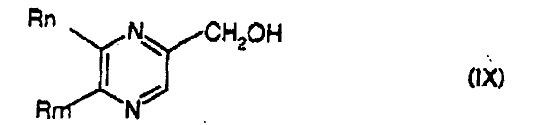 in which one of the substituents Rm or Rn represents a -CH₂OH radical and the other represents a hydrogen atom, is reacted with a derivative Hal-alk-O-alk (X) for which alk represents an alkyl radical and Hal represents a halogen atom and preferably a chlorine or bromine atom, and the product is isolated and optionally converted into a salt with an inorganic or organic acid.

13. Use of a compound of formula 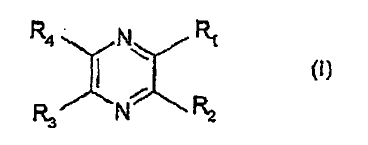 in which
R₁ represents a radical -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ in which n is equal to 0, 1, 2, 3 or 4, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ in which y is equal to 0, 1, 2, 3 or 4, -CH₂-N(CH(CH₂OH)₂)₂ or -CH₂-N(CH₂-CH(CH₂OH)₂)₂ and one of the substituents R₃ or R₄ represents a hydrogen atom and the other is identical to R₁ and R₂ represents a hydrogen atom,
R₅ represents a hydrogen atom or an alkyl radical,
R₆ represents a radical -CH₂-(CHOH)m-CH₂OH in which m is equal to 0, 1, 2, 3 or 4, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ or -CH(CH₂OH)-(CHOH)ₓ-CH₂OH in which x is equal to 1, 2, 3 or 4,
alk represents an alkyl radical,
the alkyl radicals containing, unless otherwise indicated, 1 to 6 carbon atoms in a straight or branched chain, their stereoisomers and their salts with an inorganic or organic acid, for the preparation of a medicament which is useful for the treatment or prevention of diabetes and complications of diabetes.

## Patentansprüche

1. Arzneimittel, enthaltend als Wirkstoff zumindest eine Verbindung der Formel 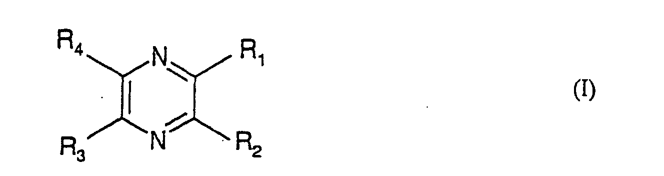 worin
R₁ einen Rest -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, worin n für 0, 1, 2, 3 oder 4 steht, -CH₂-O-R₆, -NR₅-CO-R₆, -CH₂-NR₅R₆, -CH₂-N(CH₂OH)₂, -CH₂N(CH₂-(CHOH)_{y}-CH₂OH)₂, worin y für 0, 1, 2, 3 oder 4 steht, -CH₂-N(CH(CH₂OH)₂)₂ oder -CH₂-N(CH₂-CH(CH₂OH)₂)₂ bedeutet, und einer der Substituenten R₃ oder R₄ ein Wasserstoffatom wiedergibt und der andere mit R₁ identisch ist, und R₂ für ein Wasserstoffatom bedeutet,
R₅ ein Wasserstoffatom oder einen Alkylrest bedeutet,
R₆ einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ oder -CH(CH₂OH)-(CHOH)ₓ-CH₂OH bedeutet, worin x für 1, 2, 3 oder 4 steht,
alk einen Alkylrest wiedergibt,
wobei die Alkylreste, sofern nicht anders angegeben, 1 bis 6 Kohlenstoffatome in gerader oder verzweigter Kette enthalten, deren Stereoisomere und deren Salze mit einer Mineralsäure oder organischen Säure.

2. Arzneimittel, enthaltend eine Verbindung der Formel (I) gemäß Anspruch 1, ausgewählt unter den folgenden Verbindungen:
- N,N'-Bis-(2-hydroxyethyl)-pyrazin-2,5-dicarboxamid
-N,N'-Bis-(2-hydroxyethyl)-pyrazin-2,6-dicarboxamid
- N-Methyl-N-(2-hydroxyethyl)-N'-methyl-N'-(2-hydroxyethyl)-pyrazin-2,5-dicarboxamid
- N-Methyl-N-(2-hydroxyethyl)-N'-methyl-N'-(2-hydroxyethyl)-pyrazin-2,6-dicarboxamid
- N,N,N',N'-Tetrakis-(2-hydroxyethyl)-pyrazin-2,5-dicarboxamid
- N,N,N',N'-Tetrakis-(2-hydroxyethyl)-pyrazin-2,6-dicarboxamid
- N,N'-Bis-(2,3-dihydroxyprop-1-yl)-pyrazin-2,5-dicarboxamid
- N,N'-Bis-(2,3-Dihydroxyprop-1-yl)-pyrazin-2,6-dicarboxamid
- N-Methyl-N-(2,3-dihydroxyprop-1-yl)-N'-methyl-N'-(2,3-dihydroxyprop-1-yl)-pyrazin-2,5-dicarboxamid
- N-Methyl-N-(2,3-dihydroxyprop-1-yl)-N'-methyl-N'-(2,3-dihydroxyprop-1-yl)-pyrazin-2,6-dicarboxamid
- N,N,N',N'-Tetrakis-(2,3-dihydroxyprop-1-yl)-pyrazin-2,5-dicarboxamid
- N,N,N',N'-Tetrakis-(2,3-dihydroxyprop-1-yl)-pyrazin-2,6-dicarboxamid
- N,N'-Bis-(1,3-dihydroxyprop-2-yl)-pyrazin-2,5-dicarboxamid
- N,N'-Bis-(1,3-dihydroxyprop-2-yl)-pyrazin-2,6-dicarboxamid
- N-Methyl-N-(1,3-dihydroxyprop-2-yl)-N'-methyl-N'-(1,3-dihydroxyprop-2-yl)-pyrazin-2,5-dicarboxamid
- N-Methyl-N-(1,3-dihydroxyprop-2-yl)-N'-methyl-N'-(1,3-dihydroxyprop-2-yl)-pyrazin-2,6-dicarboxamid
- N,N,N',N'-Tetrakis-(1,3-dihydroxyprop-2-yl)-pyrazin-2,5-dicarboxamid
- N,N,N',N'-Tetrakis-(1,3-dihydroxyprop-2-yl)-pyrazin-2,6-dicarboxamid
- N,N'-Bis-(2,3,4-trihydroxybut-1-yl)-pyrazin-2,5-dicarboxamid
- N,N'-Bis-(2,3,4-trihydroxybut-1-yl)-pyrazin-2,6-dicarboxamid
- N-Methyl-N-(2,3,4-trihydroxybut-1-yl)-N'-methyl-N'-(2,3,4―trihydroxybut-1-yl)-pyrazin-2,5-dicarboxamid
- N-Methyl-N-(2,3,4-trihydroxybut-1-yl)-N'-methyl-N'-(2,3,4-trihydroxybut-1-yl)-pyrazin-2,6-dicarboxamid
- N,N'-Bis-(1,3,4-trihydroxybut-2-yl)-pyrazin-2,5-dicarboxamid
- N,N'-Bis-(1,3,4-trihydroxybut-2-yl)-pyrazin-2,6-dicarboxamid
- N-Methyl-N-(1,3,4-trihydroxybut-2-yl)-N'-methyl-N'-(1,3,4-trihydroxybut-2-yl)-pyrazin-2,5-dicarboxamid
-N-Methyl-N-(1,3,4-trihydroxybut-2-yl)-N'-methyl-N'-(1,3,4-trihydroxybut-2-yl)-pyrazin-2,6-dicarboxamid
- N,N'-Bis-[2-(hydroxymethyl)-3-hydroxyprop-1-yl]-pyrazin-2,5-dicarboxamid
- N,N'-Bis-[2-(hydroxymethyl)-3-hydroxyprop-1-yl]-pyrazin,2,6-dicarboxamid
- N-Methyl-N-[2-(hydroxymethyl)-3-hydroxyprop-1-yl]-N'-methyl-N'-[2-(hydroxymethyl)-3-hydroxyprop-1-yl)-pyrazin-2,5-dicarboxamid
- N-Methyl-N-[2-(hydroxymethyl)-3-hydroxyprop-1-yl]-N'-methyl-N'-[2-(hydroxymethyl]-3-hydroxyprop-1-yl]-pyrazin-2,6-dicarboxamid
- N,N,N',N'-Tetrakis-[2-(hydroxymethyl)-3-hydroxyprop-1-yl]-pyrazin-2,5-dicarboxamid
- N,N,N',N'-Tetrakis-[2-(hydroxymethyl)-3-hydroxyprop-1-yl]-pyrazin-2,6-dicarboxamid
- N,N'-Bis-[1,1,1-tri-(hydroxymethyl)-methyl]-pyrazin-2,5-dicarboxamid
- N,N'-Bis-[1,1,1-tri-(hydroxymethyl)-methyl]-pyrazin-2,6-dicarboxamid
- N-Methyl-N-[1,1,1-tri-(hydroxymethyl)-methyl]-N'-methyl-N'-[1,1,1-tri-(hydroxymethyl)-methyl]-pyrazin-2,5-dicarboxamid
- N-Methyl-N-[1,1,1-tri-(hydroxymethyl)-methyl]-N'-methyl-N'-[1,1,1-tri-(hydroxymethyl)-methyl]-pyrazin-2,6-dicarboxamid
- N,N'-Bis-[1,1-di-(hydroxymethyl)-eth-1-yl]-pyrazin-2,5-dicarboxamid
- N,N'-Bis-[1,1-di-(hydroxymethyl)-eth-1-yl]-pyrazin-2,6-dicarboxamid
- N-Methyl-N-[1,1-di-(hydroxymethyl)-eth-1-yl]-N'-methyl-N'-[1,1-di-(hydroxymethyl)-eth-1-yl]-pyrazin-2,5-dicarboxamid
- N-Methyl-N-[1,1-di-(hydroxymethyl)-eth-1-yl]-N'-methyl-N'-[1,1-di(hydroxymetyl)-eth-1-yl]-pyrazin-2,6-dicarboxamid
- N,N,N',N'-Tetrakis-(1,3-dihydroxyprop-2-yl)-pyrazin-2,5-dicarboxamid
- N,N,N',N'-Tetrakis-(1,3-dihydroxyprop-2-yl)-pyrazin-2,6-dicarboxamid
- N,N'-Bis-(2-hydroxyethyl)-pyrazin-2,5-dimethanamin
- N,N'-Bis-(2-hydroxyethyl)-pyrazin-2,6-dimethanamin
- N-Methyl-N-(2-hydroxyethyl)-N'-methyl-N'-(2-hydroxyethyl)-pyrazin-2,5-dimethanamin
- N-Methyl-N-(2-hydroxyethyl)-N'-methyl-N'-(2-hydroxyethyl)-pyrazin-2,6-dimethanamin
- N,N,N',N'-Tetrakis-(2-hydroxyethyl)-pyrazin-2,5-dimethanamin
- N,N,N',N'-Tetrakis-(2-hydroxyethyl)-pyrazin-2,6-dimethanamin
- N,N'-Bis-(2,3-dihydroxyprop-1-yl)-pyrazin-2,5-dimethanamin
- N,N'-Bis-(2,3-dihydroxyprop-1-yl)-pyrazin-2,6-dimethanamin
- N-Methyl-N-(2,3-dihydroxyprop-1-yl)-N'-methyl-N'-(2,3-dihydroxyprop-1-yl)-pyrazin-2,5-dimethanamin
- N-Methyl-N-(2,3-dihydroxyprop-1-yl)-N'-methyl-N'-(2,3-dihydroxyprop-1-yl)-pyrazin-2,6-dimethanamin
- N,N,N',N'-Tetrakis-(2,3-dihydroxyprop-1-yl)-pyrazin-2,5-dimethanamin
- N,N,N',N'-Tetrakis-(2,3-dihydroxyprop-1-yl)-pyrazin-2,6-dimethanamin
- N,N'-Bis-(1,3-dihydroxyprop-2-yl)-pyrazin-2,5-dimethanamin
- N,N'-Bis-(1,3-dihydroxyprop-2-yl)-pyrazin-2,6-dimethanamin
-N-Methyl-N-(1,3-dihydroxyprop-2-yl)-N'-methyl-N'-(1,3-dihydroxyprop-2-yl)-pyrazin-2,5-dimethanamin
- N-Methyl-N-(1,3-dihydroxyprop-2-yl)-N'-methyl-N'-(1,3-dihydroxyprop-2-yl)-pyrazin-2,6-dimethanamin
- N,N,N',N'-Tetrakis-(1,3-dihydroxyprop-2-yl)-pyrazin-2,5-dimethanamin
- N,N,N',N'-Tetrakis-(1,3-dihydroxyprop-2-yl)-pyrazin-2,6-dimethanamin
- N,N'-Bis-(2,3,4-trihydroxybut-1-yl)-pyrazin-2,5-dimethanamin
- N,N'-Bis-(2,3,4-trihydroxybut-1-yl)-pyrazin-2,6-dimethanamin
- N-Methyl-N-(2,3,4-trihydroxybut-1-yl)-N'-methyl-N'-(2,3,4-trihydroxybut-1-yl)-pyrazin-2,5-dimethanamin
- N-Methyl-N-(2,3,4-trihydroxybut-1-yl)-N'-methyl-N'-(2,3,4-trihydroxybut-1-yl)-pyrazin-2,6-dimethanamin
- N,N'-Bis-(1,3,4-trihydroxybut-2-yl)-pyrazin-2,5-dimethanamin
- N,N'-Bis-(1,3,4-trihydroxybut-2-yl)-pyrazin-2,6-dimethanamin
- N-Methyl-N-(1,3,4-trihydroxybut-2-yl)-N'-methyl-N'-(1,3,4-trihydroxybut-2-yl)-pyrazin-2,5-dimethanamin
- N-Methyl-N-(1,3,4-trihydroxybut-2-yl)-N'-methyl-N'-(1,3,4-trihydroxybut-2-yl)-pyrazin-2,6-dimethanamin
- N,N'-Bis-[2-(hydroxymethyl)-3-hydroxyprop-1-yl]-pyrazin-2,5-dimethanamin
- N,N'-Bis-[2-(hydroxymethyl)-3-hydroxyprop-1-yl]-pyrazin-2,6-dimethanamin
- N-Methyl-N-[2-(hydroxymethyl)-3-hydroxyprop-1-yl]-N'-methyl-N'-[2-(hydroxymethyl)-3-hydroxyprop-1-yl]-pyrazin-2,5-dimethanamin
- N-Methyl-N-[2-(hydroxymethyl)-3-hydroxyprop-1-yl]-N'-methyl-N'-[2-(hydroxymethyl)-3-hydroxyprop-1-yl]-pyrazin-2,6-dimethanamin
- N,N,N',N'-Tetrakis-[2-(hydroxymethyl)-3-hydroxyprop-1-yl]-pyrazin-2,5-dimethanamin
- N,N,N',N'-Tetrakis-[2-(hydroxymethyl)-3-hydroxyprop-1-yl]-pyrazin-2,6-dimethanamin
- N,N'-Bis-[1,1,1-tri-(hydroxymethyl)-methyl]-pyrazin-2,5-dimethanamin
- N,N'-Bis-[1,1,1-tri-(hydroxymethyl)-methyl]-pyrazin-2,6-dimethanamin
- N-Methyl-N-[1,1,1-tri-(hydroxymethyl)-methyl]-N'-methyl-N'-[1,1,1-tri-(hydroxymethyl)-methyl]-pyrazin-2,5-dimethanamin
- N-Methyl-N-[1,1,1-tri-(hydroxymethyl)-methyl]-N'-methyl-N'-[1,1,1-tri-(hydroxymethyl)-methyl]-pyrazin-2,6-dimethanamin
- N,N'-Bis-[1,1-di-(hydroxymethyl)-eth-1-yl]-pyrazin-2,5-dimethanamin
- N,N'-Bis-[1,1-di-(hydroxymethyl)-eth-1-yl]-pyrazin-2,6-dimethanamin
- N-Methyl-N-[1,1-di-(hydroxymethyl)-eth-1-yl]-N'-methyl-N'-[1,1-di-(hydroxymethyl)-eth-1-yl]-pyrazin-2,5-dimethanamin
- N-Methyl-N-[1,1-di-(hydroxymethyl)-eth-1-yl]-N'-methyl-N'-[1,1-di-(hydroxymethyl)-eth-1-yl]-pyrazin-2,6-dimethanamin
- 2,5-Bis-(2-hydroxyethyloxymethyl)-pyrazin
- 2,6-Bis-(2-hydroxyethyloxymethyl)-pyrazin
- 2,5-Bis-[(2R)-2,3-dihydroxyprop-1-yloxymethyl]-pyrazin
- 2,5-Bis-[(2S)-2,3-dihydroxyprop-1-yloxymethyl]-pyrazin
- 2,6-Bis-[(2R)-2,3-dihydroxyprop-1-yloxymethyl]-pyrazin
- 2,6-Bis-[(2S)-2,3-dihydroxyprop-1-yloxymethyl]-pyrazin
- 2,5-Bis-(1,3-dihydroxyprop-2-yloxymethyl)-pyrazin
- 2,6-Bis-(1,3-dihydroxyprop-2-yloxymethyl)-pyrazin
- 2,5-Bis-(2,3,4-trihydroxybut-1-yloxymethyl)-pyrazin
- 2,6-Bis-(2,3,4-trihydroxybut-1-yloxymethyl)-pyrazin
- 2,5-Bis-(1,3,4-trihydroxybut-2-yloxymethyl)-pyrazin
- 2,6-Bis-(1,3,4-trihydroxybut-2-yloxymethyl)-pyrazin
- 2,5-Bis-[2-(hydroxymethyl)-3-hydroxyprop-1-yloxymethyl]-pyrazin
- 2,6-Bis-[2-(hydroxymethyl)-3-hydroxyprop-1-yloxymethyl]-pyrazin
- 2,5-Bis-[1,1,1-tri-(hydroxymethyl)-methyloxymethyl]-pyrazin
- 2,6-Bis-[1,1,1-tri-(hydroxymethyl)-methyloxymethyl]-pyrazin
- 2,5-Bis-[1,1-di-(hydroxymethyl)-eth-1-yloxymethyl]-pyrazin
- 2,6-Bis-[1,1-di-(hydroxymethyl)-eth-1-yloxymethyl]-pyrazin
- Bis-N,N'-(2-hydroxyethylamido)-2,5-pyrazin
- Bis-N,N'-(2-hydroxyethylamido)-2,6-pyrazin
- Bis-[N-methyl-(2-hydroxyethylamido)]-2,5-pyrazin
- Bis-[N-methyl-(2-hydroxyethylamido)]-2,6-pyrazin
- Bis-(2,3-dihydroxyprop-1-ylamido)-2,5-pyrazin
- Bis-(2,3-dihydroxyprop-1-ylamido)-2,6-pyrazin
- Bis-[N-methyl-(2,3-dihydroxyprop-1-ylamido)]-2,5-pyrazin
- Bis-[N-methyl-(2,3-dihydroxyprop-1-ylamido)]-2,6-pyrazin
- Bis-(N-methyl-((1,3-dihydroxyprop-2-ylamido)-2,5-pyrazin
- Bis-(N-methyl-((1,3-dihydroxyprop-2-ylamido)-2,6-pyrazin
- Bis-(2,3,4-trihydroxybut-1-ylamido)-2,5-pyrazin
- Bis-(2,3,4-trihydroxybut-1-ylamido)-2,6-pyrazin
- Bis-[N-methyl-(2,3,4-trihydroxybut-1-ylamido)]-2,5-pyrazin
- Bis-[N-methyl-(2,3,4-trihydroxybut-1-ylamido)]-2,6-pyrazin
- Bis-(1,3,4-trihydroxybut-2-ylamido)-2,5-pyrazin
- Bis-(1,3,4-trihydroxybut-2-ylamido)-2,6-pyrazin
- Bis-[N-methyl-(1,3,4-trihydroxybut-2-ylamido)]-2,5-pyrazin
- Bis-[N-methyl-(1,3,4-trihydroxybut-2-ylamido)]-2,6-pyrazin
- Bis-[2-(hydroxymethyl)-3-hydroxyprop-1-ylamido]-2,5-pyrazin
- Bis-[2-(hydroxymethyl)-3-hydroxyprop-1-ylamido]-2,6-pyrazin
- Bis-{[N-methyl-[2-(hydroxymethyl)-3-hydroxyprop-1-ylamido]}-2,5-pyrazin
- Bis-{[N-methyl-[2-(hydroxymethyl)-3-hydroxyprop-1-ylamido]}-2,6-pyrazin
- Bis-[1,1,1-tri-(hydroxymethyl)-methylamido]-2,5-pyrazin
- Bis-[1,1,1-tri-(hydroxymethyl)-methylamido]-2,6-pyrazin
- Bis-{[N-methyl-[1,1,1-tri-(hydroxymethyl)-methylylamido]}-2,5-pyrazin
- Bis-{[N-methyl-[1,1,1-tri-(hydroxymethyl)-methylylamido]}-2,6-pyrazin
- Bis-[1,1-di-(hydroxymethyl)-eth-1-ylamido]-2,5-pyrazin
- Bis-[1,1-di-(hydroxymethyl)-eth-1-ylamido]-2,6-pyrazin
- Bis-{[N-methyl-[1,1-di-(hydroxymethyl)-eth-1-ylamido]}-2,5-pyrazin
-Bis-{[N-methyl-[1,1-di-(hydroxymethyl)-eth-1-ylamido]}-2,6-pyrazin
oder ein Stereoisomeres dieser Verbindungen oder ein Salz einer derartigen Verbindung mit einer pharmazeutisch annehmbaren Mineralsäure oder organischen Säure.

3. Arzneimittel, enthaltend als Wirkstoff zumindest eine Verbindung der Formel (I) gemäß Anspruch 1, worin
R₁ einen Rest -CO-NR₅R₆, -CO-N(CH₂-(CHOH)ₙ-CH₂OH)₂, worin n für 0, 1, 2, 3 oder 4 steht, -CH₂-NR₅R₆, -CH₂-N(CH₂-(CHOH)_{y}-CH₂OH)₂, worin y für 0, 1, 2, 3 oder 4 steht, bedeutet und einer der Substituenten R₃ oder R₄ ein Wasserstoffatom wiedergibt und der andere mit R₁ identisch ist, R₂ ein Wasserstoffatom bedeutet, R₅ ein Wasserstoffatom oder einen Alkylrest bedeutet und R₆ einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃ bedeutet, und
alk einen Alkylrest wiedergibt
wobei die Alkylreste, sofern nicht anders angegeben, 1 bis 6 Kohlenstoffatome in gerader oder verzweigter Kette enthalten, deren Stereoisomere und deren Salze mit einer Mineralsäure oder organischen Säure.

4. Arzneimittel, enthalten als Wirkstoff eine Verbindung der Formel (I) gemäß Anspruch 1, ausgewählt unter den folgenden Verbindungen:_
- N,N'-Bis-[(tris-(hydroxymethyl)-methyl]-pyrazin-2,5-dicarboxamid
- N-Methyl-N-[(2S,3R,4R,5R)-2,3,4,5-tetrahydroxyhex-1-yl]-N'-methyl-N'-[(2S,3R,4R,5R)-2,3,4,5-tetrahydroxyhex-1-yl]-pyrazin-2,5-dicarboxamid
- N,N'-Bis-(1,3-dihydroxyprop-2-yl)-pyrazin-2,5-dicarboxamid
- N,N'-Bis-(2,3-dihydroxyprop-1-yl)-pyrazin-2,5-dicarboxamid
- N,N,N',N'-Tetrakis-(2-hydroxyethyl)-pyrazin-2,5-dicarboxamid
- Bis-{N-[2(R,S)-3-dihydroxyprop-1-yl]-N-methyl)-pyrazin-2,5-dicarboxamid
- N,N'-Bis-(2-methoxyethyl)-pyrazin-2,5-dicarboxamid
- N,N'-Bis-(2-hydroxyethyloxyethyl)-pyrazin-2,5-dicarboxamid
- N,N'-Bis-(1,3-dihydroxyprop-2-yl)-pyrazin-2,5-dimethanamin
- N,N,N',N'-Tetrakis-(2-hydroxyethyl)-pyrazin-2,5-dimethanamin
- N,N'-Bis-(1,3-dihydroxyprop-2-yl)-pyrazin-2,5-dimethanamin
- N-Methyl-N-[(2S,3R,4R,5R)-2,3,4,5-Tetrahydroxyhex-1-yl]-N'-methyl-N'-[(2S,3R,4R,5R)-2,3,4,5-tetrahydroxyhex-1-yl]-pyrazin-2,5-dimethanamin
oder ein Salz einer derartigen Verbindung mit einer pharmazeutisch annehmbaren Mineralsäure oder organischen Säure.

5. Verbindungen der Formel: 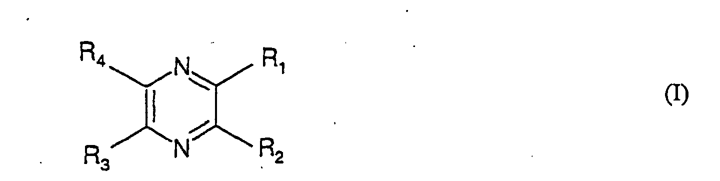 worin R₁ einen Rest -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, worin n für 0, 1, 2, 3 oder 4 steht, -CH₂-O-R₆, -NR₅-CO-R₆, -CH₂-NR₅R₆, -CH₂-N(CH₂OH)₂, -CH₂N(CH₂-(CHOH)_{y}-CH₂OH)₂, worin y für 0, 1, 2, 3 oder 4 steht, -CH₂-N(CH(CH₂OH)₂)₂ oder -CH₂-N(CH₂-CH(CH₂OH)₂)₂ bedeutet, und einer der Substituenten R₃ oder R₄ ein Wasserstoffatom wiedergibt und der andere mit R₁ identisch ist, und R₂ ein Wasserstoffatom bedeutet,
R₅ ein Wasserstoffatom oder einen Alkylrest bedeutet,
R₆ einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ oder -CH(CH₂OH)(CHOH)ₓ-CH₂OH, worin x für 1, 2, 3 oder 4 steht, bedeutet,
alk einen Alkylrest wiedergibt,
wobei die Alkylreste, sofern nicht anders angegeben, 1 bis 6 Kohlenstoffatome in gerader oder verzweigter Kette enthalten, deren Stereoisomere und deren Salze mit einer Mineralsäure oder organischen Säure.

6. Verbindungen der Formel (I) gemäß Anspruch 5, worin
R₁ einen Rest -CO-NR₅R₆, -CO-N(CH₂-(CHOH)ₙ-CH₂OH)₂, worin n für 0, 1, 2, 3 oder 4 steht, -CH₂-NR₅R₆, -CH₂-N(CH₂-(CHOH)_{y}-CH₂OH)₂, worin y für 0, 1, 2, 3 oder 4 steht, bedeutet und einer der Substituenten R₃ oder R₄ ein Wasserstoffatom bedeutet und der andere mit R₁ identisch ist, R₂ ein Wasserstoffatom bedeutet, R₅ ein Wasserstoffatom oder einen Alkylrest bedeutet, und R₆ einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃ bedeutet,
alk einen Alkylrest wiedergibt,
die Alkylreste, sofern nicht anders angegeben, 1 bis 6 Kohlenstoffatome in gerader oder verzweigter Kette enthalten,
deren Stereoisomere und deren Salze mit einer pharmazeutisch annehmbaren Mineralsäure oder organischen Säure.

7. Verbindungen der Formel (I) gemäß Anspruch 5, ausgewählt unter den folgenden Verbindungen:
- N,N'-Bis-[(tris-(hydroxymethyl)-methyl]-pyrazin-2,5-dicarboxamid
- N-Methyl-N-[(2S,3R,4R,5R)2,3,4,5-tetrahydroxyhex-1-yl]-N'-methyl-N'-[(2S,3R,4R,5R)-2,3,4,5-tetrahydroxyhex-1-yl]-pyrazin-2,5-dicarboxamid
- N,N'-Bis-(1,3-dihydroxyprop-2-yl)-pyrazin-2,5-dicarboxamid
- N,N'-Bis-(2,3-dihydroxyprop-1-yl)-pyrazin-2,5-dicarboxamid
- N,N,N',N'-Tetrakis-(2-hydroxyethyl)-pyrazin-2,5-dicarboxamid
- Bis-{N-[2-(R,S)-3-dihydroxyprop-1-yl]-N-methyl}-pyrazin-2,5-dicarboxamid
- N,N'-Bis-(2-methoxyethyl)-pyrazin-2,5-dicarboxamid
- N,N'-Bis-(2-hydroxyethyloxyethyl)-pyrazin-2,5-dicarboxamid
- N,N'-Bis-(1,3-dihydroxyprop-2-yl)-pyridin-2,6-dicarboxamid
- N,N'-Bis-(1,3-dihydroxyprop-2-yl)-pyrazin-2,5-dimethanamin
- N,N,N',N'-Tetrakis-(2-hydroxyethyl)-pyrazin-2,5-dimethanamin
- N,N'-Bis-(1,3-dihydroxyprop-2-yl)-pyrazin-2,5-dimethanamin
- N-Methyl-N-[(2S,3R,4R,5R)-2,3,4,5-tetrahydroxyhex-1-yl]-N'-methyl-N'-[(2S,3R,4R,5R)-2,3,4,5-tetrahydroxyhex-1-yl]-pyrazin-2,5-dimethanamin
und deren Salze mit einer Mineralsäure oder organischen Säure.

8. Verfahren zur Herstellung der Verbindungen der Formel (I) gemäß Anspruch 5, worin R₁ einen Rest -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂ bedeutet und einer der Substituenten R₃ und R₄ ein Wasserstoffatom wiedergibt und der andere mit R₁ identisch ist und R₂ ein Wasserstoffatom bedeutet, **dadurch gekennzeichnet, dass** man ein Derivat der Formel 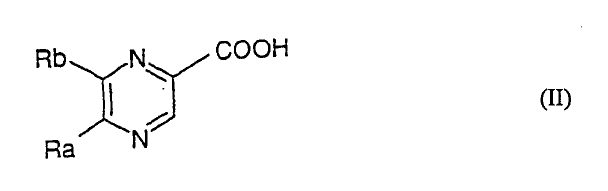 worin einer der Substituenten Ra oder Rb einen Carboxyrest und der andere ein Wasserstoffatom bedeutet, oder ein reaktives Derivat dieser Disäure mit einem Amin HNRcRd (III) umsetzt, worin entweder Rc ein Wasserstoffatom oder einen Alkylrest bedeutet und Rd einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ oder -CH(CH₂OH)(CHOH)ₓ-CH₂OH, worin x für 1, 2, 3 oder 4 steht und alk einen Alkylrest wiedergibt, bedeutet, oder Rc und Rd identisch sind und jeweils einen Rest -CH₂-(CHOH)ₙ-CH₂OH, worin n für 0, 1, 2, 3 oder 4 steht, -CH(CH₂OH)₂, -CH₂OH oder -CH₂-CH(CH₂OH)₂ bedeuten, wobei die Alkylreste 1 bis 6 Kohlenstoffatome in gerader oder verzweigter Kette enthalten, das Produkt isoliert und es gegebenenfalls mit einer Mineralsäure oder organischen Säure in ein Salz überführt.

9. Verfahren zur Herstellung der Verbindungen der Formel (I) gemäß Anspruch 5, worin R₁ einen Rest -CH₂-O-R₆ bedeutet und einer der Substituenten R₃ oder R₄ ein Wasserstoffatom bedeutet und der andere mit R₁ identisch ist und R₂ ein Wasserstoffatom bedeutet, **dadurch gekennzeichnet, dass** man ein Derivat der Formel 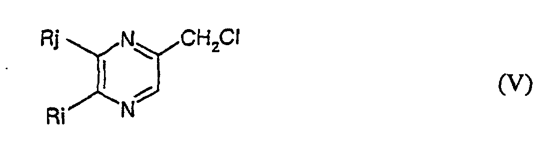 worin einer der Substituenten Ri oder Rj einen Rest -CH₂Cl und der andere ein Wasserstoffatom bedeutet, mit einem Derivat R₆OH (VI) umsetzt, worin R₆ die in Anspruch 1 angegebenen Bedeutungen besitzt, das Produkt isoliert und es gegebenenfalls mit einer Mineralsäure oder organischen Säure in ein Salz überführt.

10. Verfahren zur Herstellung der Verbindungen der Formel (I) gemäß Anspruch 5, worin R₁ einen Rest -CH₂-NR₅R₆, -CH₂-N(CH₂OH)₂, -CH₂-N(CH₂-(CHOH)_{y}-CH₂OH)₂,-CH₂-N(CH(CH₂OH)₂)₂ oder -CH₂-N(CH₂-CH(CH₂OH)₂)₂ bedeutet und einer der Substituenten R₃ oder R₄ ein Wasserstoffatom bedeutet und der andere mit R₁ identisch ist und R₂ ein Wasserstoffatom bedeutet, **dadurch gekennzeichnet, dass** man ein Derivat der Formel 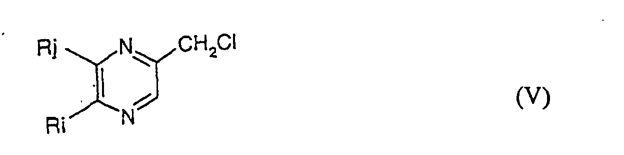 worin einer der Substituenten Ri und Rj einen Rest -CH₂Cl und der andere ein Wasserstofatom bedeutet, mit einem Amin HNRcRd (III) umsetzt, worin entweder Rc ein Wasserstoffatom oder einen Alkylrest bedeutet, und Rd einen Rest -CH₂-(CHOH)m-CH₂OH, worin m für 0, 1, 2, 3 oder 4 steht, -alk-O-alk-CH₂OH, -alk-O-alk, -CH(CH₂OH)₂,-C(CH₂OH)₃, -C(CH₃)(CH₂OH)₂, -CH₂-CH(CH₂OH)₂ oder -CH(CH₂OH)(CHOH)ₓ-CH₂OH, worin x für 1, 2, 3 oder 4 steht, bedeutet, oder Rc und Rd identisch sind und einen Rest -CH₂OH, -CH₂-(CHOH)y-CH₂OH, worin y für 0, 1, 2, 3 oder 4 steht oder -CH₂-CH(CH₂OH)₂ bedeuten, das Produkt isoliert und es gegebenenfalls mit einer Mineralsäure oder organischen Säure in ein Salz überführt.

11. Verfahren zur Herstellung der Verbindungen der Formel (I) gemäß Anspruch 5, worin R₁ ein Rest -NR₅-CO-R₆ bedeutet und einer der Substituenten R₃ oder R₄ ein Wasserstoffatom bedeutet und der andere mit R₁ identisch ist und R₂ ein Wasserstoffatom bedeutet, **dadurch gekennzeichnet, dass** man ein Derivat der Formel: 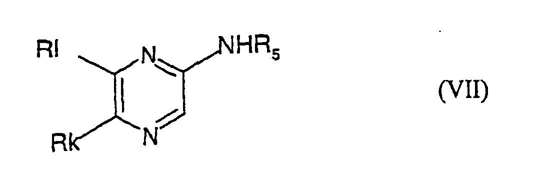 worin R₅ die in Anspruch 1 angegebenen Bedeutungen besitzt und einer der Substituenten Rk oder Rl ein Wasserstoffatom und der andere einen Rest NHR₅, worin R₅ die in Anspruch 1 angegebenen Bedeutungen besitzt, bedeutet, mit einem Derivat HOOC-R₆ (VIII), worin R₆ die in Anspruch 1 angegebenen Bedeutungen besitzt oder einem reaktiven Derivat dieser Säure umsetzt, das Produkt isoliert und es gegebenenfalls mit einer Mineralsäure oder organischen Säure in ein Salz überführt.

12. Verfahren zur Herstellung der Verbindungen der Formel (I) gemäß Anspruch 5, worin R₁ einen Rest -CH₂O-R₆ bedeutet, worin R₆ einen Rest -alk-O-alk bedeutet und einer der Substituenten R₃ oder R₄ ein Wasserstoffatom bedeutet und der andere mit R₁ identisch ist und R₂ ein Wasserstoffatom bedeutet, **dadurch gekennzeichnet, dass** man ein Derivat der Formel: 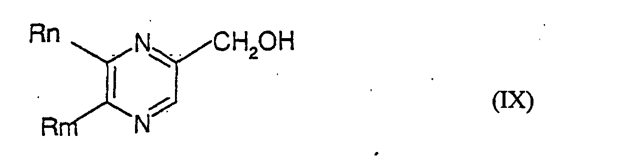 worin einer der Substituenten Rm oder Rn einen Rest -CH₂OH und der andere ein Wasserstoffatom bedeutet, mit einem Derivat Hal-alk-O-alk (X) umsetzt, worin alk einen Alkylrest bedeutet und Hal für ein Halogenatom und vorzugsweise für ein Chlor- oder Bromatom steht, das Produkt isoliert und es gegebenenfalls mit einer Mineralsäure oder organischen Säure in ein Salz überführt.

13. Verwendung einer Verbindung der Formel 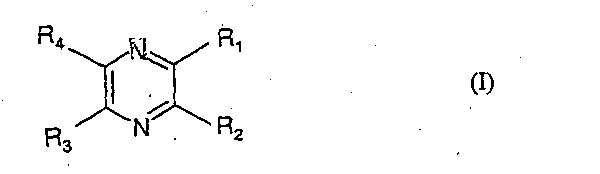 worin
R₁ einen Rest -CO-NR₅R₆, -CO-N(CH(CH₂OH)₂)₂, -CO-N(CH₂OH)₂, -CO-N(CH₂-CH(CH₂OH)₂)₂, -CO-N(CH₂-(CHOH)ₙ-CH₂OH)₂, worin n für 0, 1, 2, 3 oder 4 steht, -CH₂-O-R₆, -NR₅-CO-R₆, -CH₂-NR₅R₆, -CH₂-N(CH₂OH)₂, -CH₂-N(CH₂-(CHOH)_{y}-CH₂OH)₂ worin y für 0, 1, 2, 3 oder 4 steht, -CH₂-N(CH(CH₂OH)₂)₂ oder -CH₂-N(CH₂-CH(CH₂OH)₂)₂ bedeutet, und einer der Substituenten R₃ oder R₄ ein Wasserstoffatom bedeutet und der andere mit R₁ identisch ist und R₂ ein Wasserstoffatom bedeutet,
R₅ ein Wasserstoffatom oder einen Alkylrest bedeutet,
R₆ einen Rest -CH₂-(CHOH)m-CH₂OH, worin m für 0, 1, 2, 3 oder 4 steht, -alk-O-alk-CH₂OH, -alk-O-alk, -CH(CH₂OH)₂, -C(CH₂OH)₃, -C(CH₃)(CH₂OH)₂, -CH₂-CH(CH₂OH)₂ oder -CH(CH₂OH)-(CHOH)ₓ-CH₂OH, worin x für 1, 2, 3 oder 4 steht, bedeutet,
alk einen Alkylrest wiedergibt,
wobei die Alkylreste, sofern nicht anders angegeben, 1 bis 6 Kohlenstoffatome in gerader oder verzweigter Kette enthalten, von deren Stereoisomeren und deren Salzen mit einer Mineralsäure oder organischen Säure für die Herstellung eines Arzneimittels zur Behandlung oder Prävention von Diabetes und Diabetes-Komplikationen.
